(19) **Europäisches Patentamt / European Patent Office / Office européen des brevets**

(11) **EP 4 491 731 A1**

(12) **EUROPEAN PATENT APPLICATION**
published in accordance with Art. 153(4) EPC

(43) Date of publication:
**15.01.2025 Bulletin 2025/03**

(21) Application number: **23766954.4**

(22) Date of filing: **09.03.2023**

(51) International Patent Classification (IPC):
*C12N 15/57* (2006.01)   *C12N 1/15* (2006.01)
*C12N 1/19* (2006.01)   *C12N 1/21* (2006.01)
*C12N 5/10* (2006.01)   *C12N 9/52* (2006.01)
*C12N 9/80* (2006.01)   *C12N 15/63* (2006.01)
*C12P 1/00* (2006.01)

(52) Cooperative Patent Classification (CPC):
**C12N 5/10; C12N 9/48; C12N 9/52; C12N 9/80; C12N 15/63; C12N 15/74; C12N 15/80; C12N 15/81; C12P 1/00**

(86) International application number:
**PCT/JP2023/009183**

(87) International publication number:
**WO 2023/171778 (14.09.2023 Gazette 2023/37)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:  **09.03.2022   JP 2022036683
26.04.2022   JP 2022072469
18.05.2022   JP 2022081567
27.05.2022   JP 2022087170
22.06.2022   JP 2022100227
29.07.2022   JP 2022122327**

(71) Applicant: **Amano Enzyme Inc.
Nagoya-shi
Aichi 460-8630 (JP)**

(72) Inventors:
• **TAKAHASHI, Tetsuya**
  **Kakamigahara-shi, Gifu 509-0109 (JP)**
• **ONO, Atsushi**
  **Kakamigahara-shi, Gifu 509-0109 (JP)**
• **YOSHIDA, Kazunori**
  **Kakamigahara-shi, Gifu 509-0109 (JP)**

(74) Representative: **Müller-Boré & Partner
Patentanwälte PartG mbB
Friedenheimer Brücke 21
80639 München (DE)**

Remarks:
The complete document including Reference Table(s) and the Sequence Listing(s) can be downloaded from the EPO website

(54) **MODIFIED PROTEIN GLUTAMINASE**

(57)   The purpose of the present invention is to provide a protein glutaminase in which a decrease in reactivity due to a high temperature is suppressed. This protein glutaminase is formed of a polypeptide having an amino acid sequence obtained by introducing a substitution of a predetermined amino acid residue or amino acid sequence to an amino acid residue or an amino acid sequence at a specific location in the amino acid sequence indicated in SEQ ID NO: 1. In the protein glutaminase, a decrease in reactivity at a high temperature is suppressed more as compared to the polypeptide having the amino acid sequence indicated by SEQ ID NO: 1.

**Description**

Technical Field

The present invention relates to a modified protein glutaminase. More specifically, the present invention relates to a protein glutaminase modified so as to suppress a decrease in reactivity at a high temperature.

Background Art

Protein glutaminase is an enzyme that acts on protein being a polymer, and catalyzes a reaction in which an amide group-containing side chain is decomposed (i.e., deamidated) without cleavage of peptide bonds and crosslinking of the protein. Protein glutaminase deamidates glutamine residues in protein to generate negatively charged carboxyl groups, so that the protein undergoes various characteristic changes. For example, an increase in hydration force and a rise in electrostatic repulsion due to a decrease in isoelectric point of protein leads to a decrease in interaction between proteins (i.e., a decrease in association property), and thus enhances the solubility and water dispersibility of the protein. In addition, the exposure of inner hydrophobic regions due to a change in higher order structure of protein imparts interfacial activity to the protein, and thus improves the emulsifying capacity, emulsion stability, foaming property, and foam stability of the protein. Protein glutaminase can significantly change the properties of protein as described above, and therefore have dramatically expanded the uses of protein. For this reason, protein glutaminase is very useful, and has attracted much attention in the art.

Protein glutaminase was first discovered in Chryseobacterium proteolyticum in 2000 (Non-Patent Literature 1). Since then, protein glutaminase derived from C. proteolyticum has been industrially used as the only active ingredient of protein glutaminase enzyme agents for a long time.

Citation List

Non Patent Literature

NPL 1: A novel protein-deamidating enzyme from Chryseobacterium proteolyticum sp. nov., a newly isolated bacterium from soil. Applied and Environmental Microbiology 2000; 66(8): 3337-43

Summary of Invention

Technical Problem

Protein glutaminase derived from C. proteolyticum, for which the optimal temperature is as high as 60°C, thus is very useful, but has significantly decreased reactivity at a temperature higher than the optimal temperature. If there is a significant difference between the enzyme reactivity at an optimal temperature and that at a higher temperature as described above, there may be difficult to control the amount of an enzyme to be added in the case where the actual treatment temperature can change within the relevant temperature zone. Therefore, in the present situation, when protein glutaminase derived from C. proteolyticum is used, temperature conditions should be strictly restricted with consideration given to the intrinsic thermal properties of the enzyme.

On the other hand, in view of the possibility of further expansion of the uses of protein glutaminase, which is expected from its high usefulness, it is desirable to suppress the decrease in reactivity as much as possible and exploit the potential of protein glutaminase as much as possible even if the actual treatment temperature is higher than the optimal temperature for protein glutaminase derived from C. proteolyticum.

Accordingly, an object of the present invention is to provide a protein glutaminase in which a decrease in reactivity at a high temperature is suppressed. Solution to Problem

The present inventors have extensively conducted studies, and resultantly found a new mutation that can suppress a decrease in reactivity at a high temperature in protein glutaminase as compared to that in wild-type protein glutaminase (specifically, improve the relative activity of protein glutaminase at 65°C with respect to the reactivity of protein glutaminase at 60°C as compared with the relative activity of wild-type protein glutaminase). The present invention has been completed on the basis of these findings. That is, the present invention provides inventions of aspects as listed below.

Item 1. A modified protein glutaminase including one of the following polypeptides (I) to (III):

(I) a polypeptide consisting of an amino acid sequence obtained by introducing at least one of

(A) a substitution of the amino acid residue at position 38 with an alanine residue, a phenylalanine residue, an isoleucine residue, a leucine residue, a methionine residue, an arginine residue, a valine residue, a tyrosine residue, a cysteine residue, a lysine residue, a serine residue, or a threonine residue,

(B) a substitution of the amino acid residue at position 156 with a tyrosine residue,

(C) a substitution of the four consecutive amino acid residues at positions 72 to 75 with an amino acid sequence consisting of RYGVKMSNQDG (SEQ ID NO: 2),

(D) a substitution of the amino acid residue at position 64 with a glutamic acid residue,

(E) a substitution of the amino acid residue at position 82 with a proline residue, a tryptophan residue, or a phenylalanine residue,

(F) a substitution of the amino acid residue at position 116 with an isoleucine residue, a valine residue, a cysteine residue, a leucine residue, or a serine residue,

(G) a substitution of the amino acid residue at position 140 with an isoleucine residue, a glutamic acid residue, a tyrosine residue, an aspartic acid residue or a valine residue,

(H) a substitution of the amino acid residue at position 2 with an arginine residue or a tryptophan residue,

(I) a substitution of the amino acid residue at position 3 with an arginine residue,

(J) a substitution of the amino acid residue at position 16 with an alanine residue, a cysteine residue, a phenylalanine residue, a leucine residue, a methionine residue, an arginine residue, a serine residue, a valine residue, or a tryptophan residue,

(K) a substitution of the amino acid residue at position 86 with a proline residue,

(L) a substitution of the amino acid residue at position 5 with a leucine residue, a threonine residue, or a valine residue,

(M) a substitution of the amino acid residue at position 9 with a glutamic acid residue, an aspartic acid residue, a methionine residue, a cysteine residue, a phenylalanine residue, a glycine residue, a proline residue, an arginine residue, or a tryptophan residue,

(N) a substitution of the amino acid residue at position 11 with a cysteine residue, a glycine residue, a histidine residue, a methionine residue, an arginine residue, or a valine residue,

(O) a substitution of the amino acid residue at position 13 with a methionine residue, an asparagine residue, a phenylalanine residue, or a tryptophan residue,

(P) a substitution of the amino acid residue at position 17 with a glycine residue, or an alanine residue,

(Q) a substitution of the amino acid residue at position 19 with a leucine residue, a methionine residue, an arginine residue, a serine residue, or a threonine residue,

(R) a substitution of the amino acid residue at position 20 with a glycine residue, a glutamic acid residue, or a serine residue,

(S) a substitution of the amino acid residue at position 22 with a glycine residue,

(T) a substitution of the amino acid residue at position 26 with a valine residue,

(U) a substitution of the amino acid residue at position 30 with a cysteine residue, a phenylalanine residue, a histidine residue, an arginine residue, a valine residue, a tyrosine residue, or a glutamine residue,

(V) a substitution of the amino acid residue at position 50 with a tryptophan residue,

(W) a substitution of the amino acid residue at position 51 with a proline residue, a histidine residue, a tryptophan residue, or a phenylalanine residue,

(X) a substitution of the amino acid residue at position 52 with a valine residue,

(Y) a substitution of the amino acid residue at position 53 with a glutamine residue,

(Z) a substitution of the amino acid residue at position 54 with an asparagine residue,

(AA) a substitution of the amino acid residue at position 57 with a cysteine residue,

(AB) a substitution of the amino acid residue at position 58 with a threonine residue,

(AD) a substitution of the amino acid residue at position 73 with a valine residue,

(AE) a substitution of the three consecutive amino acid residues at positions 73 to 75 with an amino acid sequence consisting of a PMG,

(AF) a substitution of the amino acid residue at position 79 with a lysine residue or an arginine residue,

(AH) a substitution of the amino acid residue at position 92 with a glycine residue, a serine residue, or a valine residue,

(AI) a substitution of the amino acid residue at position 94 with a tryptophan residue, or a tyrosine residue,

(AJ) a substitution of the amino acid residue at position 95 with an aspartic acid residue, a phenylalanine residue, a cysteine residue, or a methionine residue,

(AK) a substitution of the amino acid residue at position 96 with an alanine residue, a phenylalanine residue, a glycine residue, an isoleucine residue, a lysine residue, a leucine residue, an asparagine residue, a glutamine residue, an arginine residue, a serine residue, a threonine residue, a tryptophan residue, a tyrosine residue, an aspartic acid residue, a cysteine residue, or a methionine residue,

(AL) a substitution of the amino acid residue at position 97 with a lysine residue,

(AM) a substitution of the amino acid residue at position 160 with an aspartic acid residue, or a phenylalanine residue,

(AN) a substitution of the amino acid residue at position 164 with an alanine residue, a cysteine residue, an aspartic acid residue, a phenylalanine residue, a histidine residue, a leucine residue, a methionine residue, an arginine residue, or a serine residue,

(AO) a substitution of the amino acid residue at position 12 with a cysteine residue, a methionine residue, a tryptophan residue, or a tyrosine residue,

(AP) a substitution of the amino acid residue at position 24 with a serine residue,

(AQ) a substitution of the amino acid residue at position 98 with a phenylalanine residue,

(AR) a substitution of the amino acid residue at position 131 with a leucine residue,

(AS) a substitution of the amino acid residue at position 134 with a tryptophan residue,

(AT) a substitution of the amino acid residue at position 135 with an isoleucine residue, a cysteine residue, a phenylalanine residue, or a valine residue,

(AU) a substitution of the amino acid residue at position 136 with an isoleucine residue, an arginine residue, a leucine residue, or a valine residue,

(AV) a substitution of the amino acid residue at position 168 with an arginine residue, an asparagine residue, or a cysteine residue,

(AW) a substitution of the amino acid residue at position 175 with a threonine residue, an alanine residue, a valine residue, or a cysteine residue,

(AX) a substitution of the amino acid residue at position 182 with a leucine residue, a methionine residue, a glutamic acid residue, a glutamine residue, a glycine residue, an aspartic acid residue, or an alanine residue, and

(AY) a substitution of the amino acid residue at position 184 with a tryptophan residue, or a phenylalanine residue

into the amino acid sequence set forth as SEQ ID NO: 1; (II) a polypeptide in which one or several amino acid residues other than the substituted amino acid residues are substituted, added, inserted or deleted in the amino acid sequence in which at least one of the substitutions (A) to (AY) is introduced, and the relative activity of protein glutaminase at 65°C with respect to the activity of protein glutaminase at 60°C is improved as compared to the relative activity of the polypeptide consisting of the amino acid sequence set forth as SEQ ID NO: 1; and

(III) a polypeptide in which the sequence identity of regions that do not include the substituted amino acid residues is 70% or more in the amino acid sequence in which at least one of the substitutions (A) to (AY) is introduced, and the relative activity of protein glutaminase at 65°C with respect to the activity of protein glutaminase at 60°C is improved as compared to the relative activity of the polypeptide consisting of the amino acid sequence set forth as SEQ ID NO: 1.

Item 2. A DNA encoding the modified protein glutaminase according to item 1.

Item 3. An expression cassette or a recombinant vector including the DNA according to item 2.

Item 4. A transformant obtained by transforming a host using the expression cassette or recombinant vector according to item 3.

Item 5. A method for producing a modified protein glutaminase, including the step of culturing the transformant according to item 4.

Item 6. An enzyme agent including the modified protein glutaminase according to item 1.

Item 7. A modifier for a protein material, including the modified protein glutaminase according to item 1.

Item 8. A method for producing a modified protein material, including the step of applying the modified protein glutaminase according to item 1 to a protein material. Advantageous Effect of Invention

According to the present invention, a modified protein glutaminase in which a decrease in reactivity at a high temperature is suppressed is provided.

Brief Description of Drawing

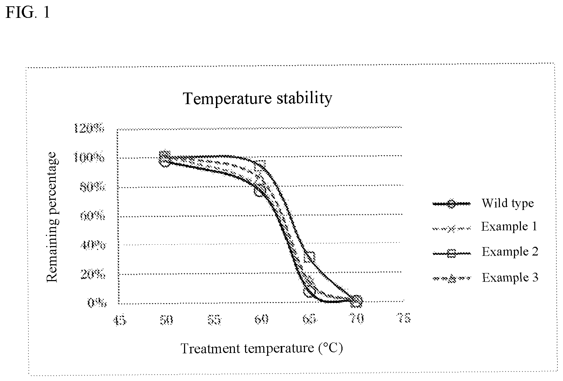

[Fig. 1] Fig. 1 shows the results of confirming the temperature stability of the modified protein glutaminases of Examples 1 to 3 which are obtained in Test Example 1.

Description of Embodiments

Hereinafter, the present invention will be described in detail. The 20 types of amino acid residues in the amino acid sequence may be represented by one character in abbreviation. Specifically, glycine (Gly) is G, alanine (Ala) is A, valine (Val) is V, leucine (Leu) is L, isoleucine (Ile) is I, phenylalanine (Phe) is F, tyrosine (Tyr) is Y, tryptophan (Trp) is W, serine (Ser) is S, threonine (Thr) is T, cysteine (Cys) is C, methionine (Met) is M, aspartic acid (Asp) is D, glutamic acid (Glu) is E, asparagine (Asn) is N, glutamine (Gln) is Q, lysine (Lys) is K, arginine (Arg) is R, histidine (His) is H, and proline (Pro) is P.

In the amino acid sequence described herein, the left end is a N-terminus, and the right end is a C-terminus.

The term "non-polar amino acid", as used herein, includes glycine, alanine, valine, leucine, isoleucine, proline, methionine, phenylalanine, and tryptophan. The term "non-charged amino acids", as used herein, includes glycine, serine, threonine, cysteine, tyrosine, asparagine, and glutamine. The "acidic amino acid" includes aspartic acid and glutamic acid. The "basic amino acid" includes lysine, arginine, and histidine.

The term "substitution", as used herein, includes not only a case where a substitution of an amino acid residue is artificially introduced, but also a case where a substitution of an amino acid residue is naturally introduced, that is, a case where amino acid residues are intrinsically different. The substitution of an amino acid residue, as used herein, may be an artificial substitution or a natural substitution, and is preferably an artificial substitution.

1. Modified protein glutaminase

The modified protein glutaminase of the present invention includes one of the following polypeptides (I) to (III).

(I) A polypeptide consisting of an amino acid sequence obtained by introducing at least one of the substitutions (A) to (AY) shown in the tables below into the amino acid sequence set forth as SEQ ID NO: 1.

[Table 1A]

|  |  | Position of substitution in SEQ ID NO: 1 | Amino acid residue or amino acid sequence after substitution at position of substitution in left column |
|---|---|---|---|
| (A) | | Position 38 | A, F, I, L, M, R, V, Y, C, K, SorT |
| (B) | | Position 156 | Y |
| (C) | | Positions 72 to 75 | Sequence RYGVKMSNQDG (SEQ ID NO: 2) |
| (D) | | Position 64 | E |
| (E) | | Position 82 | P, W or F |
| (F) | | Position 116 | I, V, C, L or S |
| (G) | | Position 140 | I, E, Y, D or V |
| (H) | | Position 2 | RorW |
| (I) | | Position 3 | R |
| (J) | | Position 16 | A, C, F, L, M, R, S, VorW |
| (K) | | Position 86 | P |
| (L) | | Position 5 | L, TorV |
| (M) | | Position 9 | E, D, M, C, F, G, P, RorW |
| (N) | | Position 11 | C, G, H, M, RorV |
| (O) | | Position 13 | M, N, F or W |
| (P) | | Position 17 | GorA |
| (Q) | | Position 19 | L, M, R, S or T |
| (R) | | Position 20 | G, E or S |
| (S) | | Position 22 | G |
| (T) | | Position 26 | V |
| (U) | | Position 30 | C, F, H, R, V, Y or Q |
| (V) | | Position 50 | W |

(continued)

|  | Position of substitution in SEQ ID NO: 1 | Amino acid residue or amino acid sequence after substitution at position of substitution in left column |
|---|---|---|
| (W) | Position 51 | P, H, W or F |
| (X) | Position 52 | V |
| (Y) | Position 53 | Q |
| (Z) | Position 54 | N |

[Table 1B]

|  | Position of substitution in SEQ ID NO: 1 | Amino acid residue or amino acid sequence after substitution at position of substitution in left column |
|---|---|---|
| (AA) | Position 57 | C |
| (AB) | Position 58 | T |
| (AD) | Position 73 | V |
| (AE) | Positions 73 to 75 | Sequence PMG |
| (AF) | Position 79 | KorR |
| (AH) | Position 92 | G, SorV |
| (AI) | Position 94 | WorY |
| (AJ) | Position 95 | D, F, C or M |
| (AK) | Position 96 | A, F, G, I, K, L, N, Q, R, S, T, W, Y, D, C or M |
| (AL) | Position 97 | K |
| (AM) | Position 160 | D or F |
| (AN) | Position 164 | A, C, D, F, H, L, M, R or S |
| (AO) | Position 12 | C, M, W or Y |
| (AP) | Position 24 | S |
| (AQ) | Position 98 | F |
| (AR) | Position 131 | L |
| (AS) | Position 134 | W |
| (AT) | Position 135 | I, C, ForV |
| (AU) | Position 136 | I, R, L or V |
| (AV) | Position 168 | R, N or C |
| (AW) | Position 175 | T, A, V or C |
| (AX) | Position 182 | L, M, E, Q, G, D or A |
| (AY) | Position 184 | WorF |

(II) a polypeptide in which one or several amino acid residues other than the substituted amino acid residues are substituted, added, inserted or deleted in the amino acid sequence in which at least one of the substitutions (A) to (AY) is introduced, and the relative activity of protein glutaminase at 65°C with respect to the activity of protein glutaminase at 60°C is improved as compared to the relative activity of the polypeptide consisting of the amino acid sequence set forth as SEQ ID NO: 1; and

(III) a polypeptide in which the sequence identity of regions that do not include the substituted amino acid residues is 70% or more in the amino acid sequence in which at least one of the substitutions (A) to (AY) is introduced, and the relative activity of protein glutaminase at 65°C with respect to the activity of protein glutaminase at 60°C is improved as compared to the relative activity of the polypeptide consisting of the amino acid sequence set forth as SEQ ID NO: 1.

The amino acid sequence set forth as SEQ ID NO: 1 is a mature sequence from the full-length sequence (sequence including signal sequence and pro-sequence) of protein glutaminase derived from Chryseobacterium proteolyticum, which is set forth as SEQ ID NO: 3.

Although the sequence of the polypeptide (I) is so evident that it is not necessary to show its specific example, for example, the specific amino acid sequence of the polypeptide having a substitution with an alanine residue, as the substitution (A), is set forth as SEQ ID NO: 4, the specific amino acid sequence of the polypeptide having the substitution (B) is set forth as SEQ ID NO: 5, and the specific amino acid sequence of the polypeptide having the substitution (C) is set forth as SEQ ID NO: 6. The specific amino acid sequences of polypeptides having substitutions (D) to (AY) are similarly established.

Specific examples of the polypeptides (II) and (III) include polypeptides obtained by introducing the substitution into protein glutaminase analogous to protein glutaminase derived from C. proteolyticum. Examples of the analogous protein glutaminase into which the substitution is introduced include protein glutaminase derived from Chryseobacterium sp., more specifically an amino acid sequence set forth as SEQ ID NO: 7 or SEQ ID NO: 8, which is a mature sequence of protein glutaminase derived from Chryseobacterium sp., or an amino acid sequence of SEQ ID NO: 7 or SEQ ID NO: 8 in which the amino acid residue at position 115 is substituted with another amino acid residue (for example, a serine residue). SEQ ID NO: 7 has a sequence identity of 86.9% with SEQ ID NO: 1 and SEQ ID NO: 8 has a sequence identity of 87.4% with SEQ ID NO: 1. The full-length sequence of SEQ ID NO: 7 is the amino acid sequence set forth as SEQ ID NO: 9, and the full-length sequence of SEQ ID NO: 8 is the amino acid sequence set forth as SEQ ID NO: 10.

In the polypeptide (II), the amino acid modification introduced may include, among substitution, addition, insertion, and deletion, only one type of modification (for example, only substitution) or two or more types of modifications (for example, substitution and insertion). In the polypeptide (II), the number of amino acid differences at an arbitrary difference site may be 1 or several numbers, and is, for example, 1 to 18, and preferably 1 to 10.

More preferred examples of the polypeptide (II) include the following (II-1) and (II-2).

(II-1) A polypeptide in which 1 to 9, 1 to 8, 1 to 7, 1 to 6, 1 to 5 or 1 to 4, more preferably 1 to 3, and particularly preferably 1 or 2, or 1 amino acid residues other than the substituted amino acid residues are substituted, added, inserted or deleted in the amino acid sequence obtained by introducing at least one of the substitutions (A) to (AY) into the amino acid sequence set forth as SEQ ID NO: 1, and the relative activity of protein glutaminase at 65°C with respect to the activity of protein glutaminase at 60°C is improved as compared to the relative activity of the polypeptide consisting of the amino acid sequence set forth as SEQ ID NO: 1.

(II-2) A polypeptide of the amino acid sequence obtained by introducing at least one of the substitutions (A) to (AY) into the amino acid sequence set forth as SEQ ID NO: 8, and amino acid sequence in which 1 to 9, 1 to 8, 1 to 7, 1 to 6, 1 to 5 or 1 to 4, more preferably 1 to 3, and particularly preferably 1 or 2, or 1 amino acid residues other than the substituted amino acid residues are substituted, added, inserted or deleted in the amino acid sequence obtained by introducing at least one of the substitutions (A) to (AY) into the amino acid sequence set forth as SEQ ID NO: 8 and the relative activity of protein glutaminase at 65°C with respect to the activity of protein glutaminase at 60°C is improved as compared to the relative activity of the polypeptide consisting of the amino acid sequence set forth as SEQ ID NO: 8.

In the polypeptide (III), the sequence identity with the amino acid sequence set forth as SEQ ID NO: 1 may be 70% or more, and is preferably 80% or more, or 85% or more, and more preferably 90% or more, or 93% or more.

Still more preferred examples of the polypeptide (III) include the following (III-1) and (III-2).

(III-1) A polypeptide in which the sequence identity of regions that do not include the substituted amino acid residues is 95% or more, more preferably 98% or more, still more preferably 98.5% or more, or 99%, particularly preferably 99.3% or more, or 99.5% or more in the amino acid sequence obtained by introducing at least one of the substitutions (A) to (AY) into the amino acid sequence set forth as SEQ ID NO: 1, and the relative activity of protein glutaminase at 65°C with respect to the activity of protein glutaminase at 60°C is improved as compared to the relative activity of the polypeptide consisting of the amino acid sequence set forth as SEQ ID NO: 1.

(III-2) A polypeptide of the amino acid sequence obtained by introducing at least one of the substitutions (A) to (AY) into the amino acid sequence set forth as SEQ ID NO: 8, and amino acid sequence in which the sequence identity of regions that do not include the substituted amino acid residues is 95% or more, more preferably 98% or more, still more preferably 98.5% or more, or 99%, particularly preferably 99.3% or more, or 99.5% or more in the amino acid sequence obtained by introducing at least one of the substitutions (A) to (AY) into the amino acid sequence set forth as SEQ ID NO: 8 and the relative activity of protein glutaminase at 65°C with respect to the activity of protein glutaminase at 60°C is improved as compared to the relative activity of the polypeptide consisting of the amino acid sequence set forth as SEQ ID NO: 8.

Here, in the polypeptide (III), the sequence identity with each amino acid sequence set forth as SEQ ID NO: 1 or 8 is sequence identity calculated by comparison with the amino acid sequence set forth as SEQ ID NO: 1 or 8. The "sequence identity" indicates a value of identity of an amino acid sequence obtained by the bl2seq program (Tatiana A. Tatsusova, Thomas L. Madden, FEMS Microbiol. Lett., Vol. 174, p247-250, 1999) of BLASTPACKAGE [sgi32 bit edition, Version 2.0.12; available from National Center for Biotechnology Information (NCBI)]. The parameters may be set to Gap insertion Cost value: 11 and Gap extension Cost value: 1.

In the polypeptides of (II) and (III), amino acid residues corresponding to position 42 (cysteine residue), position 83 (histidine residue) and position 103 (aspartic acid residue) in the amino acid sequence set forth as SEQ ID NO: 1 or 8 may be active catalyst residues, and therefore it is desirable not to introduce substitutions or deletions at these sites.

When an amino acid substitution is introduced to SEQ ID NO: 1 or 8 in the polypeptides (II) and (III), a preferred type of the amino acid substitution introduced includes conservative substitution. That is, for example, the substitution in the polypeptides (II) and (III) is such that when the amino acid before substitution is a non-polar amino acid, a substitution with another non-polar amino acid is introduced, or when the amino acid before substitution is a non-charged amino acid, a substitution with another non-charged amino acid is introduced, or when the amino acid before substitution is an acidic amino acid, a substitution with another acidic-polar amino acid is introduced, or when the amino acid before substitution is a basic amino acid, a substitution with another basic amino acid is introduced.

The polypeptides (I) to (III) above have protein glutaminase activity, and the thermal property that a decrease in reactivity at a high temperature is suppressed. The term "thermal property that a decrease in reactivity at a high temperature is suppressed" means that the relative value of the activity of protein glutaminase at 65°C with respect to the activity of protein glutaminase at 60°C is improved (hereinafter, also referred to as "relative activity $A_{65°C/60°C}$") as compared to the relative activity $A_{65°C/60°C}$ of the polypeptide consisting of the amino acid sequence set forth as SEQ ID NO: 1 (wild-type protein glutaminase derived from Chryseobacterium proteolyticum), specifically, the relative activity $A_{65°C/60°C}$ is 1.1 times or more of the relative activity $A_{65°C/60°C}$ of wild-type protein glutaminase which is the polypeptide consisting of the amino acid sequence shown in SEQ ID NO: 1. The relative activity $A_{65°C/60°C}$ of the polypeptides (II-2) and (III-2) above is 1.05 times or more of the relative activity $A_{65°C/60°C}$ of the polypeptide consisting of the amino acid sequence set forth as SEQ ID NO: 8 (wild-type protein glutaminase derived from Chryseobacterium sp.). As a preferred example of the thermal property of the polypeptides (I) to (III) above, the relative activity $A_{65°C/60°C}$ is 1.15 times or more, more preferably 1.2 times or more, still more preferably 1.25 times or more, even more preferably 1.3 times or more, furthermore preferably 1.4 times or more, and particularly preferably 1.5 times or more of the relative activity $A_{65°C/60°C}$ of wild-type protein glutaminase which is a polypeptide consisting of the amino acid sequence set forth as SEQ ID NO: 1. The upper limit of the relative activity $A_{65°C/60°C}$ of the polypeptides (I) to (III) above is not limited, and for example, is 2 times or less or 1.8 times or less of the relative activity $A_{65°C/60°C}$ of wild-type protein glutaminase which is the polypeptide consisting of the amino acid sequence set forth as SEQ ID NO: 1.

When presented in terms of the relative value of the activity of protein glutaminase at 65°C with respect to the activity of protein glutaminase at 57°C (hereinafter, also referred to as relative activity $B_{65°C/57°C}$), the thermal property that a decrease in reactivity at a high temperature is suppressed in the polypeptides (I) to (III) is, for example, 1.03 times or more, preferably 1.05 times or more, more preferably 1.1 times or more, or 1.2 times or more, still more preferably 1.3 times or more, or 1.4 times or more, even more preferably 1.7 times or more, furthermore preferably 2 times or more, and particularly preferably 2.3 times or more of the relative activity $B_{65°C/57°C}$ of wild-type protein glutaminase which is the polypeptide consisting of the amino acid sequence set forth as SEQ ID NO: 1. The upper limit of the relative activity $B_{65°C/57°C}$ of the polypeptides (I) to (III) above is not limited, and for example, is 3 times or less or 2.5 times or less of the relative activity $B_{65°C/57°C}$ of wild-type protein glutaminase which is the polypeptide consisting of the amino acid sequence set forth as SEQ ID NO: 1.

The specific activity of the polypeptides (I) to (III) is not limited, and the specific activity of the polypeptides (I) to (III) at 37°C is, for example, 20% or more, 40% or more, or 60% or more when the specific activity of the polypeptide consisting of the amino acid sequence set forth as SEQ ID NO: 1 (wild-type protein glutaminase derived from Chryseobacterium proteolyticum) at 37°C is defined as 100%, or the specific activity of the polypeptides (I) to (III) at 60°C is, for example, 20% or more, 40% or more, or 60% or more when the specific activity of the polypeptide consisting of the amino acid sequence set forth as SEQ ID NO: 1 (wild-type protein glutaminase derived from Chryseobacterium proteolyticum) at 60°C is defined as 100%. The specific activity of the polypeptides (I) to (III) is preferably equivalent to the specific activity of the polypeptide consisting of the amino acid sequence set forth as SEQ ID NO: 1 (wild-type protein glutaminase derived from Chryseobacterium proteolyticum). The equivalent specific activity, specifically the specific activity of the polypeptides (I) to (III) at 37°C when the specific activity, at 37°C, of the polypeptide consisting of the amino acid sequence set forth as SEQ ID NO: 1 is defined as 100% is 80% or more, preferably 90% or more, more preferably 95% or more, still more preferably 100% or more, and even more preferably 105% or more, or the specific activity of the polypeptides (I) to (III) at 60°C when the specific activity, at 60°C, of the polypeptide consisting of the amino acid sequence set forth as SEQ ID NO: 1 is defined as 100% is 80% or more, preferably 90% or more, more preferably 95% or more, still more preferably 100% or more, and even more preferably 105% or more.

The amount of an enzyme producing ammonia at 1 μmol per minute using Z-Gln-Gly (benzyloxycarbonyl-L-glutaminylglycine) as a substrate is defined as one unit (1 U) of the protein glutaminase activity.

The modified protein glutaminase of the present invention may be used as an active ingredient of an enzyme agent described later, or may be used for forming a part of a larger protein integrated in the form of a fusion protein or the like with peptides or proteins consisting of other amino acid sequences (hereinafter, also referred to as "other proteins etc."). Examples of the other proteins etc. include peptides used for purification of proteins, such as a polyhistidine residue, and derived from an additional sequence for securing stability of mRNA during recombinant production.

2. DNA

The DNA of the present invention is DNA encoding the modified protein glutaminase described in "1. Modified protein glutaminase" above.

The DNA of the present invention is not limited as long as it has a nucleotide sequence encoding a modified protein glutaminase including any of the polypeptides (I) to (III) described in "1. Modified protein glutaminase" above. Examples of the nucleotide sequence of DNA encoding the amino acid sequence set forth as SEQ ID NO: 1 (protein glutaminase derived from Chryseobacterium proteolyticum), which is a reference sequence for the polypeptides (I) to (III), include SEQ ID NO: 11. Therefore, those skilled in the art can appropriately design the DNA of the present invention using SEQ ID NO: 11 as a reference sequence.

Examples of the DNA of the present invention include the following DNAs [i] to [iii].

[i] A DNA consisting of a nucleotide sequence obtained by introducing at least one of the substitutions (a) to (ay) shown in the tables below into the nucleotide sequence set forth as SEQ ID NO: 11.

[Table 2A]

| <1> | <2> Position of substitution in SEQ ID NO: 11 | <3> Amino acid or amino acid sequence encoded by nucleotide sequence after substitution at position of substitution in left column |
|---|---|---|
| [a] | Positions 112 to 114 | A, F, I, L, M, R, V, Y, C, K, SorT |
| [b] | Positions 466 to 468 | Y |
| [c] | Positions 214 to 225 | Sequence RYGVKMSNQDG (SEQ ID NO: 2) |
| [d] | Positions 190 to 192 | E |
| [e] | Positions 244 to 246 | P, W or F |
| [f] | Positions 346 to 348 | I, V, C, L or S |
| [g] | Positions 418 to 420 | I, E, Y, D or V |
| [h] | Positions 4 to 6 | RorW |
| [i] | Positions 7 to 9 | R |
| [j] | Positions 46 to 48 | A, C, F, L, M, R, S, VorW |
| [k] | Positions 256 to 258 | P |
| [l] | Positions 13 to 15 | L, TorV |
| [m] | Positions 25 to 27 | E, D, M, C, F, G, P, RorW |
| [n] | Positions 31 to 33 | C, G, H, M, R or V |
| [o] | Positions 37 to 39 | M, N, F or W |
| [p] | Positions 49 to 51 | GorA |
| [q] | Positions 55 to 57 | L, M, R, S or T |
| [r] | Positions 58 to 60 | G, E or S |
| [s] | Positions 64 to 66 | G |
| [t] | Positions 76 to 78 | V |
| [u] | Positions 88 to 90 | C, F, H, R, V, Y or Q |
| [v] | Positions 148 to 150 | W |
| [w] | Positions 151 to 153 | P, H, W or F |
| [x] | Positions 154 to 156 | V |

(continued)

| <1> | <2> Position of substitution in SEQ ID NO: 11 | <3> Amino acid or amino acid sequence encoded by nucleotide sequence after substitution at position of substitution in left column |
|---|---|---|
| [y] | Positions 157 to 159 | Q |
| [z] | Positions 160 to 162 | N |

[Table 2B]

| <1> | <2> Position of substitution in SEQ ID NO: 11 | <3> Amino acid or amino acid sequence encoded by nucleotide sequence after substitution at position of substitution in left column |
|---|---|---|
| [aa] | Positions 169 to 171 | C |
| [ab] | Positions 172 to 174 | T |
| [ad] | Positions 217 to 219 | V |
| [ae] | Positions 217 to 225 | Sequence PMG |
| [af] | Positions 235 to 237 | K or R |
| [ah] | Positions 274 to 276 | G, SorV |
| [ai] | Positions 280 to 282 | WorY |
| [aj] | Positions 283 to 285 | D, F, C or M |
| [ak] | Positions 286 to 288 | A, F, G, I, K, L, N, Q, R, S, T, W, Y, D, C or M |
| [al] | Positions 289 to 291 | K |
| [am] | Positions 478 to 480 | D or F |
| [an] | Positions 490 to 492 | A, C, D, F, H, L, M, R or S |
| [ao] | Positions 34 to 36 | C, M, WorY |
| [ap] | Positions 70 to 72 | S |
| [aq] | Positions 292 to 294 | F |
| [ar] | Positions 391 to 393 | L |
| [as] | Positions 400 to 402 | W |
| [at] | Positions 403 to 405 | I, C, F or V |
| [au] | Positions 406 to 408 | I, R, L or V |
| [av] | Positions 502 to 504 | R, N or C |
| [aw] | Positions 523 to 525 | T, A, V or C |
| [ax] | Positions 544 to 546 | L, M, E, Q, G, D or A |
| [ay] | Positions 550 to 552 | WorF |

Specifically, a DNA consisting of a nucleotide sequence in which at least one of the following substitutions is introduced:

[a] a substitution at positions 112 to 114 with a nucleotide sequence encoding an alanine residue, a phenylalanine residue, an isoleucine residue, a leucine residue, a methionine residue, an arginine residue, a valine residue, a tyrosine residue, a cysteine residue, a lysine residue, a serine residue, or a threonine residue,
[b] a substitution at positions 466 to 468 with a nucleotide sequence encoding a tyrosine residue,
[c] a substitution at positions 214 to 225 with a nucleotide sequence encoding the amino acid sequence set forth as SEQ ID NO: 2,
[d] a substitution at positions 190 to 192 with a nucleotide sequence encoding a glutamic acid residue,
[e] a substitution at positions 244 to 246 with a nucleotide sequence encoding a proline residue, a tryptophan residue, or a phenylalanine residue,

[f] a substitution at positions 346 to 348 with a nucleotide sequence encoding an isoleucine residue, a valine residue, a cysteine residue, a leucine residue, or a serine residue,

[g] a substitution at positions 418 to 420 with a nucleotide sequence encoding an isoleucine residue, a glutamic acid residue, a tyrosine residue, an aspartic acid residue, or a valine residue,

[h] a substitution at positions 4 to 6 with a nucleotide sequence encoding an arginine residue, or a tryptophan residue,

[i] a substitution at positions 7 to 9 with a nucleotide sequence encoding an arginine residue,

[j] a substitution at positions 46 to 48 with a nucleotide sequence encoding an alanine residue, a cysteine residue, a phenylalanine residue, a leucine residue, a methionine residue, an arginine residue, a serine residue, a valine residue, or a tryptophan residue,

[k] a substitution at positions 256 to 258 with a nucleotide sequence encoding a proline residue,

[l] a substitution at positions 13 to 15 with a nucleotide sequence encoding a leucine residue, a threonine residue, or a valine residue,

[m] a substitution at positions 25 to 27 with a nucleotide sequence encoding a glutamic acid residue, an aspartic acid residue, a methionine residue, a cysteine residue, a phenylalanine residue, a glycine residue, a proline residue, an arginine residue, or a tryptophan residue,

[n] a substitution at positions 31 to 33 with a nucleotide sequence encoding a cysteine residue, a glycine residue, a histidine residue, a methionine residue, an arginine residue, or a valine residue,

[o] a substitution at positions 37 to 39 with a nucleotide sequence encoding a methionine residue, an asparagine residue, a phenylalanine residue, or a tryptophan residue,

[p] a substitution at positions 49 to 51 with a nucleotide sequence encoding a glycine residue or an alanine residue,

[q] a substitution at positions 55 to 57 with a nucleotide sequence encoding a leucine residue, a methionine residue, an arginine residue, a serine residue, or a threonine residue,

[r] a substitution at positions 58 to 60 with a nucleotide sequence encoding a glycine residue, a glutamic acid residue, or a serine residue,

[s] a substitution at positions 64 to 66 with a nucleotide sequence encoding a glycine residue,

[t] a substitution at positions 76 to 78 with a nucleotide sequence encoding a valine residue,

[u] a substitution at positions 88 to 90 with a nucleotide sequence encoding a cysteine residue, a phenylalanine residue, a histidine residue, an arginine residue, a valine residue, a tyrosine residue, or a glutamine residue,

[v] a substitution at positions 148 to 150 with a nucleotide sequence encoding a tryptophan residue,

[w] a substitution at positions 151 to 153 with a nucleotide sequence encoding a proline residue, a histidine residue, a tryptophan residue, or a phenylalanine residue,

[x] a substitution at positions 154 to 156 with a nucleotide sequence encoding a valine residue,

[y] a substitution at positions 157 to 159 with a nucleotide sequence encoding a glutamine residue,

[z] a substitution at positions 160 to 162 with a nucleotide sequence encoding an asparagine residue,

[aa] a substitution at positions 169 to 171 with a nucleotide sequence encoding a cysteine residue,

[ab] a substitution at positions 172 to 174 with a nucleotide sequence encoding a threonine residue,

[ad] a substitution at positions 217 to 219 with a nucleotide sequence encoding a valine residue,

[ae] a substitution at positions 217 to 225 with a nucleotide sequence encoding an amino acid sequence consisting of PMG,

[af] a substitution at positions 235 to 237 with a nucleotide sequence encoding a lysine residue or an arginine residue,

[ah] a substitution at positions 274 to 276 with a nucleotide sequence encoding a glycine residue, a serine residue, or a valine residue,

[ai] a substitution at positions 280 to 282 with a nucleotide sequence encoding a tryptophan residue or a tyrosine residue,

[aj] a substitution at positions 283 to 285 with a nucleotide sequence encoding an aspartic acid residue, a phenylalanine residue, a cysteine residue, or a methionine residue,

[ak] a substitution at positions 286 to 288 with a nucleotide sequence encoding an alanine residue, a phenylalanine residue, a glycine residue, an isoleucine residue, a lysine residue, a leucine residue, an asparagine residue, a glutamine residue, an arginine residue, a serine residue, a threonine residue, a tryptophan residue, a tyrosine residue, an aspartic acid residue, a cysteine residue, or a methionine residue,

[al] a substitution at positions 289 to 291 with a nucleotide sequence encoding a lysine residue,

[am] a substitution at positions 478 to 480 with a nucleotide sequence encoding an aspartic acid residue or a phenylalanine residue,

[an] a substitution at positions 490 to 492 with a nucleotide sequence having an alanine residue, a cysteine residue, an aspartic acid residue, a phenylalanine residue, a histidine residue, a leucine residue, a methionine residue, an arginine residue, or a serine residue,

[ao] a substitution at positions 34 to 36 with a nucleotide sequence encoding a cysteine residue, a methionine residue, a tryptophan residue, or a tyrosine residue,

[ap] a substitution at positions 70 to 72 with a nucleotide sequence encoding a serine residue,

[aq] a substitution at positions 292 to 294 with a nucleotide sequence encoding a phenylalanine residue,

[ar] a substitution at positions 391 to 393 with a nucleotide sequence encoding a leucine residue,

[as] a substitution at positions 400 to 402 with a nucleotide sequence encoding a tryptophan residue,

[at] a substitution at positions 403 to 405 with a nucleotide sequence encoding an isoleucine residue, a cysteine residue, a phenylalanine residue, or a valine residue,

[au] a substitution at positions 406 to 408 with a nucleotide sequence encoding an isoleucine residue, an arginine residue, a leucine residue, or a valine residue,

[av] a substitution at positions 502 to 504 with a nucleotide sequence encoding an arginine residue, an asparagine residue, or a cysteine residue,

[aw] a substitution at positions 523 to 525 with a nucleotide sequence encoding a threonine residue, an alanine residue, a valine residue, or a cysteine residue,

[ax] a substitution at positions 544 to 546 with a nucleotide sequence encoding a leucine residue, a methionine residue, a glutamic acid residue, a glutamine residue, a glycine residue, an aspartic acid residue, or an alanine residue, and

[ay] a substitution at position 550 to position 552 with a nucleotide sequence encoding a tryptophan residue or a phenylalanine residue,

into the nucleotide sequence set forth as SEQ ID NO: 11.

[ii] A DNA encoding a polypeptide in which the relative activity of protein glutaminase at 65°C with respect to protein glutaminase at 60°C is improved as compared to the relative activity of the polypeptide consisting of the amino acid sequence set forth as SEQ ID NO: 1, the DNA hybridizing under stringent conditions with DNA consisting of a nucleotide sequence complementary to the DNA [i].

[iii] A DNA encoding a polypeptide in which the relative activity of protein glutaminase at 65°C with respect to protein glutaminase at 60°C is improved as compared to the relative activity of the polypeptide consisting of the amino acid sequence set forth as SEQ ID NO: 1, the DNA having a homology of 70% or more with the DNA [i].

Specific examples of the DNA [i] are easily determined by those skilled in the art on the basis of the nucleotide sequence of SEQ ID NO: 11. For example, the sequence of an optimal codon of E. coli described later can be selected as a nucleotide sequence encoding an amino acid or amino acid sequence shown in column <3> of Tables 2A and 2B, at a substitution position in SEQ ID NO: 11 which is shown in column <2> of Tables 2A and 2B, for DNA having a substitution shown in column <1> of Tables 2A and 2B. For example, the specific nucleotide sequence of DNA having, as the substitution [a], a substitution with a nucleotide sequence encoding an alanine residue is set forth as SEQ ID NO: 12 (sequence in which the nucleotide sequence set forth as SEQ ID NO: 11 is substituted at positions 112 to 114 with get encoding an alanine residue), the specific sequence of DNA having the substitution [b] is set forth as SEQ ID NO: 13 (sequence in which the nucleotide sequence set forth as SEQ ID NO: 11 is substituted at positions 466 to 468 with tac encoding a tyrosine residue), and the specific nucleotide sequence having the substitution [c] is set forth as SEQ ID NO: 14. The specific nucleotide sequence of DNA having a substitution with a nucleotide sequence encoding another substituted amino acid residue, as the substitution [a], and specific nucleotide sequences of DNAs having the substitutions [d] to [ay] can similarly sequenced.

For the DNA [ii], the term "under stringent conditions" refers to conditions in which DNA is held at 50°C to 65°C for 4 hours to one night in $6 \times$ SSC ($1 \times$ SSC consists of 0.15 M NaCl and 0.015 M sodium citrate at a pH of 7.0) containing 0.5% SDS, $5 \times$ Denhartz's [0.1% bovine serum albumin (BSA), 0.1% polyvinyl pyrrolidone, 0.1% Ficoll 400] and 100 $\mu$g/ml of salmon sperm DNA.

The hybridization under stringent conditions is performed specifically by the following method. That is, a nylon membrane on which a DNA library or a cDNA library is immobilized is prepared, and the nylon membrane is blocked at 65°C in a prehybridization solution containing $6 \times$ SSC, 0.5% SDS, $5 \times$ Denhartz's, and 100 $\mu$g/ml of salmon sperm DNA. Thereafter, each probe labeled with $^{32}$P is added to the nylon membrane, which is held overnight at 65°C. The nylon membrane is washed at room temperature for 10 minutes in $6 \times$ SSC, at room temperature for 10 minutes in $2 \times$ SSC containing 0.1% SDS, and at 45°C for 30 minutes in $0.2 \times$ SSC containing 0.1% SDS, and then subjected to autoradiography, whereby DNA hybridized specifically with the probe can be detected.

For the DNA [iii], the homology may be 70% or more, and is preferably 80% or more, or 85% or more, more preferably 90% or more, or 93% or more, still more preferably 95% or more, even more preferably 98% or more, furthermore preferably 98.5% or more, or 99% or more, and particularly preferably 99.3% or more, or 99.5% or more.

Here, the "homology" of DNA is calculated using published or marketed software with an algorithm that performs comparison using a reference sequence as a query sequence. Specifically, BLAST, FASTA, GENETYX (manufactured by GENETYX K.K.), or the like can be used, and they may be used by being set as default parameters.

The DNA of the present invention can be obtained by, for example, introducing at least one of the substitutions (a) to (ay) into DNA encoding a polypeptide consisting of the amino acid sequence set forth as SEQ ID NO: 1 (that is, protein glutaminase derived from C. proteolyticum) or its analogous protein glutaminase (as described above, specific examples include Chryseobacterium sp, more specifically a polypeptide consisting of the amino acid sequence set forth as SEQ ID NO: 7 or SEQ ID NO: 8). In addition, the DNA of the present invention can be artificially synthesized by a method of total synthesis of genes.

In the case of DNA encoding a polypeptide consisting of the amino acid sequence set forth as SEQ ID NO: 1, from a nucleic acid construct such as a plasmid in which the nucleotide sequence set forth as SEQ ID NO: 11 is incorporated, the nucleotide sequence can be acquired by a conventional method based on PCR. Together with the nucleotide sequence set forth as SEQ ID NO: 11, a sequence obtained by adding a signal sequence and pro-sequence to the 5'-terminus side of the nucleotide sequence can be incorporated into the nucleic acid construct. Examples of the sequence to which such a signal sequence and a pro-sequence are added include the nucleotide sequence set forth as SEQ ID NO: 15 and encoding the full-length sequence of protein glutaminase derived from C. proteolyticum, which is set forth as SEQ ID NO: 3, and the nucleotide sequence set forth as SEQ ID NO: 16 or SEQ ID NO: 17 and encoding the full-length sequence of protein glutaminase derived from Chryseobacterium sp. and being analogous protein glutaminase, which is set forth as SEQ ID NO: 9 or 10.

As the method for artificially modifying an amino acid sequence by introducing a mutation into a gene, known methods such as a Kunkel method and a Gapped duplex method, and a mutation introduction kit using a site-directed mutagenesis method, for example, QuikChange (trademark) Site-Directed Mutagenesis Kit (Stratagene Company), GeneTailor (trademark) Site-Directed Mutagenesis System (Invitrogen Company), TaKaRa Site-Directed Mutagenesis System (Mutan-K, Mutan-Super Express Km, etc.: Takara Bio Inc.), and the like can be used.

The DNA of the present invention includes various kinds of DNA derived from codon degeneracy. Artificial production of various kinds of DNA encoding the same amino acid sequence can be easily performed using a known genetic engineering method. For example, in genetic engineering production of protein, the expression level of a protein of interest may be low if the frequency of use of a codon used on an original gene encoding the protein is low in the host. In this a case, high expression of the protein of interest can be achieved by optimizing the frequency of codon usage for the host without changing the encoded amino acid sequence.

As an index of the frequency of codon usage, the total optimum frequency of codon usage for the host may be adopted for each codon. The optimal codon is defined as a codon, the usage frequency of which is the highest among codons corresponding to the same amino acid. The frequency of codon usage is not limited as long as it is optimized for the host, and examples of the optimal codon of E. coli include the following. F: phenylalanine (ttt), L: leucine (ctg), I: isoleucine (att), M: methionine (atg), V: valine (gtg), Y: tyrosine (tat), stop codon (taa), H: histidine (cat), Q: glutamine (cag), N: asparagine (aat), K: lysine (aaa), D: aspartic acid (gat), E: glutamic acid (gaa), S: serine (agc), P: proline (ccg), T: threonine (acc), A: alanine (gcg), C: cysteine (tgc), W: tryptophan (tgg), R: arginine (cgc), G: glycine (ggc).

The nucleotide sequence of DNA in which a mutation is introduced into a base sequence can be confirmed by sequencing by a conventional method. Specific examples of the sequencing method include a dideoxynucleotide chain termination method (Sanger et al. (1977) Proc. Natl. Acad. Sci. USA 74: 5463), and a sequence analysis method using an appropriate DNA sequencer. Examples of the method for confirming whether DNA encodes a polypeptide of interest include a method in which a sequenced nucleotide sequence is compared with an unsubstituted nucleotide sequence such as the nucleotide sequence set forth as SEQ ID NO: 11, and a method in which an amino acid sequence deduced from the sequenced nucleotide sequence is compared with an unsubstituted amino acid sequence such as the amino acid sequence set forth as SEQ ID NO: 1.

3. Expression cassette or recombinant vector

The expression cassette or recombinant vector of the present invention contains the DNA of the present invention which is described in "2. DNA" above. The expression cassette or recombinant vector of the present invention can be obtained by connecting a promoter and a terminator to the DNA of the present invention, or inserting the expression cassette of the present invention or the DNA of the present invention into the expression vector.

The expression cassette of the present invention or the recombinant vector of the present invention may contain, as a control factor, transcription elements such as an enhancer, a CCAAT box, a TATA box, or an SPI site, if necessary, in addition to the promoter and the terminator. These control factors may be operably connected to the DNA of the present invention. The term "operably connected" means that the DNA of the present invention and various control factors that regulate the DNA of the present invention are connected in a state of being operable in host cells.

As for the recombinant vector of the present invention, an expression vector for genetic recombination, which is constructed from a phage, a plasmid, or a virus capable of autonomously growing in a host, is suitable. Such an expression vector is known, and examples thereof include commercially available expression vectors such as pQE-based vectors (Qiagen Corporation), pDR540 and pRIT2T (GE Healthcare Bio-Sciences AB), and pET-based vectors (Merck KGaA). For the expression vector, an appropriate combination with host cells may be selected and used. For example, when E. coli is used as host cells, a combination of a pET-based vector and a DH5α E. coli strain, a combination of a pET-based vector and a BL21 (DE3) E. coli strain, or a combination of a pDR540 vector and a JM109 E. coli strain may be used.

4. Transformant

The transformant of the present invention is obtained by transforming a host with the expression cassette or recombinant vector of the present invention described in "3. Expression cassette or recombinant vector" above.

The host for use in production of the transformant of the present invention is not limited as long as it can undergo introduction of a gene, ensures the stability of the expression cassette or recombinant vector, and is capable of autonomously growing and expressing the trait of a gene containing the DNA of the present invention. Preferred examples thereof include bacteria belonging to the genus Escherichia such as Escherichia coli, the genus Bacillus such as Bacillus subtilis, the genus Pseudomonas such as Pseudomonasputida, and the genus Chryseobacterium such as Chryseobacterium proteolyticum; and yeast, and may also be animal cells, insect cells, plant cells and the like..

The transformant of the present invention can be obtained by introducing the expression cassette of the present invention or the recombinant vector of the present invention into a host. The place where the DNA of the present invention is introduced is not limited as long as a gene of interest can be expressed, and the DNA may be introduced onto a plasmid or a genome. Examples of the specific method for introducing the expression cassette of the present invention or the recombinant vector of the present invention include a recombinant vector method and a genome edition method. Conditions for introducing the expression cassette or recombinant vector into the host may be appropriately set according to the type of the host and the like. When the host is bacteria, for example, a method using competent cells under calcium ion treatment and an electroporation method are applicable. When the host is yeast, for example, an electroporation method, a spheroplast method, and a lithium acetate method are applicable. When the host is animal cells, for example, an electroporation method, a calcium phosphate method, and a lipofection method are applicable. When the host is insect cells, for example thereof, a calcium phosphate method, a lipofection method, and an electroporation method are applicable. When the host is plant cells, for example, an electroporation method, an agrobacterium method, a particle gun method, and a PEG method are applicable.

Whether the expression cassette of the present invention or the recombinant vector of the present invention has been incorporated into a host can be confirmed by a PCR method, a Southern hybridization method, a Northern hybridization method, or the like.

When whether the expression cassette of the present invention or the recombinant vector of the present invention has been incorporated into a host is confirmed by a PCR method, for example, genomic DNA, the expression cassette, or the recombinant vector may be separated and purified from a transformant.

For example, when the host is bacteria, the expression cassette or recombinant vector is separated and purified with a lysate obtained by lysing bacteria. As a method of lysis, for example, treatment with a lytic enzyme such as lysozyme is performed, and if necessary, a protease, other enzymes, and a surfactant such as sodium lauryl sulfate (SDS) are used in combination.

Physical crushing methods such as freeze-thaw and French press treatment may also be combined. The DNA can be separated and purified from the lysate by appropriately combining, for example, deproteinization treatment based on phenol treatment and protease treatment, ribonuclease treatment, alcohol precipitation treatment, and a commercially available kit.

DNA can be cleaved by, for example, restriction enzyme treatment under a conventional method. As the restriction enzyme, for example, a type II restriction enzyme that acts on a specific nucleotide sequence is used. The DNA and the expression cassette or expression vector are bound to each other using, for example, a DNA ligase.

Thereafter, a primer specific to the DNA of the present invention is designed using the separated and purified DNA as a template, and PCR is performed. The amplification product obtained by PCR is subjected to agarose gel electrophoresis, polyacrylamide gel electrophoresis, capillary electrophoresis, or the like, and stained with ethidium bromide, SYBR Green solution and the like. This enables the amplification product to be detected as a band, thereby confirming that transformation has occurred.

It is also possible to detect the amplification product by performing PCR using a primer labeled with a fluorescent dye or the like in advance. A method may also be utilized in which the amplification product is bound to a solid phase such as a microplate and confirm the amplification product by fluorescence, an enzymatic reaction, or the like.

5. Method for producing modified protein glutaminase

The method for producing a modified protein glutaminase according to the present invention is a method for producing an enzyme described "1. Modified protein glutaminase" above, the method including the step of culturing the transformant of the present invention. When one of the substitutions (A) to (AY) contained in the modified protein glutaminase is naturally introduced, the modified protein glutaminase can be obtained by a production method including the step of culturing microorganisms that produce the modified protein glutaminase.

The conditions for the culture may be appropriately set according to the nutritional and physiological properties of the transformant or the microorganisms, and liquid culture is preferable. In the case of industrial production, aerated and stirred culture is preferable. As nutrient sources of the medium, those required for growth of the transformant or the microorganisms can be used. The carbon source may be any consumable carbon compound, and examples thereof include glucose, sucrose, lactose, maltose, molasses, and pyruvic acid. The nitrogen source may be any consumable nitrogen compound, and examples thereof include peptone, meat extract, yeast extract, casein hydrolysate, and soybean cake alkaline extract. In addition to the carbon source and the nitrogen source, for example, phosphates, carbonates, sulfates, salts of magnesium, calcium, potassium, iron, manganese, zinc and the like, specific amino acids, specific vitamins, and the like may be used if necessary.

The culture temperature can be appropriately set as long as the transformant of the present invention or the microorganisms can grow, and the transformant or the microorganisms produce a modified protein glutaminase. The culture temperature is preferably about 15 to 37°C. The culture may be completed at an appropriate time that is judged to be a time when the modified protein glutaminase reaches the highest yield, and the culture time is usually about 12 to 48 hours.

After the transformant or the microorganisms are cultured, a method such as centrifugation is applied to the culture solution to collect the culture supernatant and/or bacterial cells. A mechanical method such as ultrasonication or French press or treatment with a lytic enzyme such as lysozyme is applied to the bacterial cells, and if necessary, an enzyme such as protease or a surfactant such as sodium lauryl sulfate (SDS) is used to solubilize the bacterial cells, whereby a water-soluble fraction containing a predetermined modified protein glutaminase can be obtained. It is also possible to secrete the expressed modified protein glutaminase into the culture solution by selecting an appropriate expression cassette or expression vector and host.

The thus-obtained water-soluble fraction containing the modified protein glutaminase may be directly subjected to purification treatment, or may be subjected to purification treatment after the modified protein glutaminase in the water-soluble fraction is concentrated. The concentration can be performed by, for example, concentration under reduced pressure, membrane concentration, salting-out treatment, or fractional precipitation with hydrophilic organic solvents (for example, methanol, ethanol, and acetone).

The purification treatment of the modified protein glutaminase can be performed by, for example, appropriately combining methods such as gel filtration, adsorption chromatography, ion-exchange chromatography, and affinity chromatography. The purified modified protein glutaminase may be powderized by lyophilization, vacuum drying, spray drying, or the like if necessary.

6. Enzymatic agent

The modified protein glutaminase can be provided in the form of an enzyme agent. Accordingly, the present invention also provides an enzyme agent containing the modified protein glutaminase described in "1. Modified protein glutaminase" above as an active ingredient.

The content of the modified protein glutaminase in the enzyme agent of the present invention is not limited, and the lower limit of the content is, for example, 1 U/g or more, preferably 10 U/g or more, more preferably 50 U/g or more, still more preferably 100 U/g or more, and particularly preferably 200 U/g or more. The upper limit of the content is, for example, 10,000 U/g or less, preferably 5,000 U/g or less, more preferably 2,000 U/g or less, still more preferably 1,000 U/g or less, and particularly preferably 800 U/g or less.

The enzyme agent of the present invention may, or is not required to, contain, in addition to the modified protein glutaminase, other components to the extent that the effect of the present invention is not affected. Examples of the other component include other enzymes other than the modified protein glutaminase, additives, and culture residues generated by the above-described production method.

Examples of the other enzyme include amylase (α-amylase, β-amylase, glucoamylase), glucosidase (α-glucosidase, β-glucosidase), galactosidase (α-galactosidase, β-galactosidase), protease (acidic protease, neutral protease, alkaline protease), peptidase (leucine peptidase, aminopeptidase), lipase, esterase, cellulase, phosphatase (acid phosphatase, alkaline phosphatase), nuclease, deaminase, oxidase, dehydrogenase, glutaminase, pectinase, catalase, dextranase, transglutaminase, protein deamidase (except for the above-described modified protein glutaminase), and pullulanase. The other enzymes may be contained alone, or in combination of two or more thereof.

Examples of the additive include an excipient, a buffer, a suspending agent, a stabilizer, a preservative, an antiseptic agent, and physiological saline. Examples of the excipient include starch, dextrin, maltose, trehalose, lactose, D-glucose, sorbitol, D-mannitol, sucrose, and glycerol. Examples of the buffer include phosphate, citrate, and acetate. Examples of the stabilizer include propylene glycol and ascorbic acid. Examples of the preservative include phenol, benzalkonium chloride, benzyl alcohol, chlorobutanol, and methylparaben. Examples of the antiseptic agent include ethanol, benzalkonium chloride, paraoxybenzoic acid, and chlorobutanol. These additives may be contained alone, or in combination of two or more thereof.

Examples of the culture residue include a component derived from a culture medium, contaminating protein, and a bacterial cell component.

The form of the enzyme agent of the present invention is not particularly limited, and examples thereof include a liquid form and a solid form (powder, granules, and the like). The enzyme agent in the above-described form can be prepared by a generally known method.

7. Modifier for protein material

The modified protein glutaminase can be used for known applications of protein glutaminase. For example, the modified protein glutaminase can be used for the purpose of modifying a protein material. Accordingly, the present invention also provides a modifier for a protein material which contains a modified protein glutaminase.

The specific aspect of the modification of a protein material is not limited as long as it is a change in property of protein which is caused by generation of a carboxyl group due to deamidation of the γ-amide group and the β-amide group of the glutamine residue and the asparagine residue of the protein. Specifically, examples of the modification of a protein material include enhancement of solubility of protein, enhancement of water dispersibility, improvement of emulsifying capacity, and emulsion stability. A specific method for using the modifier for a protein material is as described in "8. Method for producing modified protein material" below.

8. Method for producing modified protein material

As described above, the modified protein glutaminase can be used for the purpose of modifying a protein material. Accordingly, the present invention also provides a method for producing a modified protein material, including the step of applying the modified protein glutaminase to a protein material.

In the production method of the present invention, a mixture containing a protein material and a modified protein glutaminase is placed under conditions for application of the modified protein glutaminase to make a protein modifying reaction proceed.

The protein material is not particularly limited as long as it contains protein. The protein material may either one for edible use or one for non-food use. The edible protein material can be used as a food/drink or as a material for producing a food/drink. The non-food protein material can be used as a material for protein experiments, a medical material, a fiber material, a cosmetic material, or the like.

Specific examples of the protein material include a protein source itself, and a preparation obtained from a protein source by performing treatment for increasing the protein content using a known method, which are appropriately selected by those skilled in the art. Examples of the edible protein material include a preparation obtained from a food product containing plant protein as a plant protein material; and a preparation prepared from a food product containing animal protein as an animal protein material. Examples of the edible plant protein include bean protein such as soybean protein, broad bean protein, pea protein, chickpea bean protein, green bean protein and lupin bean protein; grain protein such as wheat protein, rye protein, oat protein and corn protein; and seed protein from canary seed, linseed, almond, cashew nut, hazelnut, pecan nut, macadamia nut, pistachio, walnut, brazil nut, peanut, coconut, hemp seed (industrial hemp), pili, chestnut, sesame, pine nut and the like. Examples of the edible animal protein include protein from livestock meat, fish meat, egg, and milk. Examples of the non-food protein material include albumin and globulin derived from a biological sample such as egg white or serum; and silk, and wool.

The content of the protein in the protein material is not limited, and is, for example, 30 wt% or more, 40 wt% or more, or 50 wt% or more, preferably 60 wt% or more, more preferably 70 wt% or more, and still more preferably 80 wt% or more. The upper limit of the content of the protein contained in the protein material is not limited, and is, for example, 95 wt% or less, 90 wt% or less, 85 wt% or less, 80 wt% or less, 70 wt% or less, or 60 wt% or less.

Examples of the content of the protein material in the mixture include an amount such that the concentration of protein contained in the protein material in the mixture is, for example, 0.1 wt% or more, or 0.3 wt% or more, preferably 0.7 wt% or more, and more preferably 1.4 wt% or more. The upper limit of the concentration of protein contained in the protein material in the mixture is not limited, and is, for example, 80 wt% or less, 60 wt% or less, 40 wt% or less, 30 wt% or less, 20 wt% or less, 15 wt% or less, 10 wt% or less, 8 wt% or less, 5 wt% or less, 3 wt% or less, or 2 wt% or less.

The amount of the modified protein glutaminase used is not limited, and the amount of the modified protein glutaminase used per gram of protein contained in the protein material is, for example, 0.1 U or more, preferably 0.5 U or more, more preferably 1 U or more, still more preferably 2 U or more, even more preferably 3.5 U or more, furthermore preferably 4.5 U or more. The upper limit of the amount of the modified protein glutaminase used per gram of protein contained in the protein material is not limited, and is, for example, 45 U or less, 35 U or less, 20 U or less, 10 U or less, 8 U or less, or 5.5 U or less.

The amount of the modified protein glutaminase used per gram of the protein material is, for example, 0.01 U or more, 0.05 U or more, or 0.1 U or more, preferably 0.5 U or more, more preferably 1 U or more, still more preferably 2 U or more, even more preferably 3 U or more, and furthermore preferably 4 U or more. The upper limit of the amount of the modified protein glutaminase used per gram of the protein material is not limited, and is, for example, 40 U or less, 30 U or less, 20 U or less, 10 U or less, 7 U or less, or 5 U or less.

The conditions for application of the modified protein glutaminase are appropriately determined on the basis of the optimal temperature and optimal pH for the modified protein glutaminase used.

The temperature condition among the conditions for application of the modified protein glutaminase is preferably a heating condition because a decrease in reactivity of the modified protein glutaminase at a high temperature is suppressed. The heating condition, under which the solubility of the substrate is higher than that under the non-heating condition, thus enables treatment in a reaction solution containing the substrate at a high concentration. As a specific temperature condition, the temperature is, for example, 40 to 80°C, preferably 46 to 77°C, more preferably 53 to 74°C, still more preferably 60 to 70°C, and particularly 60 to 65°C, or 63 to 67°C. Since a decrease in reactivity of the modified protein glutaminase at a high temperature is suppressed, the production method of the present invention is particularly useful when the temperature condition changes within the above temperature range.

As the pH condition among the conditions for application of the modified protein glutaminase, the pH is, for example, 2 to 12, preferably 3 to 10, and more preferably 4 to 9.

The time of applying the modified protein glutaminase is not limited, and may be appropriately determined according to the preparation scale or the like, and is, for example, 1 hour or more, preferably 8 hours or more, more preferably 16 hours or more, and still more preferably 20 hours or more. The upper limit of the range of the time is not limited, and is, for example, 40 hours or less, 30 hours or less, or 25 hours or less.

After completion of the reaction, enzyme deactivation treatment is performed, followed by cooling, and, if necessary, posttreatment is performed to obtain a modified protein material.

Examples

Hereinafter, the present invention will be described in detail by way of examples, which should not be construed as limiting the present invention.

Test Example 1

(1) Preparation of modified protein glutaminase

A sequence (SEQ ID NO: 11) encoding the amino acid sequence (SEQ ID NO: 1) of protein glutaminase derived from wild-type C. proteolyticum and introduced into pET21 vector was used as a template, into which various mutations were introduced to prepare the following modified protein glutaminases.

[Table 3]

| Example 1 | P38A mutant of SEQ ID NO: 1 (SEQ ID NO: 4) |
|---|---|
| Example 2 | L156Y mutant of SEQ ID NO: 1 (SEQ ID NO: 5) |
| Example 3 | Mutant in which S72 to T75 of SEQ ID NO. 1 is substituted with SEQ ID NO. 2 (RYGVKMSNQDG) (SEQ ID NO: 6) |
| Comparative Example 1 | K70R mutant of SEQ ID NO: 1 |
| Comparative Example 2 | N119A mutant of SEQ ID NO: 1 |

Specifically, using the following primers and PrimeSTAR Mutagenesis Basal Kit (Takara), PCR was performed by a conventional method to introduce mutations.

(Primer for P38A mutation)

Rv: 5'-AGATATgCTGTAGACGGATGTTATGCA-3' (SEQ ID NO: 18)
Fw: 5'-GTCTACAGcATATCTGAATGTGATGCA-3' (SEQ ID NO: 19)

(Primer for L156Y mutation)

Rv: 5'-AACAATtacATCAATACCAACTGTGTA-3' (SEQ ID NO: 20)
Fw: 5'-ATTGATgtaATTGTTGTCATACAGGTA-3' (SEQ ID NO: 21)

Using the following primers and a KOD-Plus-Mutagenesis Kit, PCR was performed by a conventional method to introduce a mutation.

(Primer for substitution of S72 to T75 with SEQ ID NO: 2 (RYGVKMSNQDG))

Rv: 5'-tgtcaaaccaggatggtTGCTGTGTGGCGTGGAGCTACCACG-3' (SEQ ID NO: 22)
Fw: 5'-TCTTAACGCCATAACGTGCCTTTAGGTTTCCGTATACAAAT-3' (SEQ ID NO: 23)

With each of the obtained mutated gene products, E. coli BL21 (DE3) was transformed by a conventional method to acquire gene expression vectors. The protein glutaminase sequence introduced into the gene expression vector was confirmed. The transformant was cultured with shaking in LB medium at 37°C for 16 hours. Thereafter, the transformant was transplanted to Terrific Broth, and cultured at 37°C for 4 hours, and IPTG (final concentration: 0.5 mM) was added, followed by shaking culture at 33°C for 20 hours. The bacterial cells were collected from the culture solution, and the bacterial cells were lysed with B-PER (trademark) Bacterial Cell Lysis Reagent (manufactured by Thermo Scientific) by a conventional method, and centrifuged at 15,000 rpm for 10 minutes. The centrifugal supernatant was collected as a crude enzyme solution, trypsin was added to the solution at a final concentration of 50 μg/mL, and the mixture was allowed to stand at 37°C for 1 hour, and then purified by a conventional method using TALON (registered trademark) Spin Columns (manufactured by Takara Bio Inc.), thereby obtaining an enzyme sample.

(2) Method for measuring protein glutaminase activity

N-Benzyloxycarbonyl-L-glutaminylglycine (Z-Gln-Gly; Peptide Institute, Inc.) was dissolved in a 0.2 mol/L phosphate buffer (pH 6.5) at 30 mmol/L, and the thus-prepared solution was used as a substrate solution. To a test tube, 0.1 mL of an enzyme solution whose activity was to be measured was added, and left standing in a constant-temperature water bath at $37 \pm 0.5$°C for 1 minute. Thereafter, 1 mL of a substrate solution left to stand at $37 \pm 0.5$°C for 10 minutes in advance was added, and the mixture was immediately mixed. This solution was left standing for 10 minutes to carry out an enzymatic reaction, and 1 mL of a 0.4 mol/L trichloroacetic acid solution was added to stop the enzymatic reaction. A measurement blank was prepared by adding 0.1 mL of an enzyme solution to a test tube, and adding 1 mL of a 0.4 mol/L of a trichloroacetic acid solution and 1 mL of a substrate solution in this order. A color development reaction by Ammonia-Test Wako (FUJIFILM Wako Pure Chemical Corporation) was carried out, and ammonia released by an enzyme reaction for 10 minutes was quantified by the value of absorbance at a wavelength of 630 nm. The ammonia concentration in the reaction solution was determined from a calibration curve showing the relationship between the ammonia concentration and the absorbance (630 nm), which had been prepared using an ammonia standard solution (ammonium chloride). The enzymatic activity of protein glutaminase was calculated from the following equation, where the amount of an enzyme producing ammonia at 1 μmol per minute is defined as one unit (1U). In the equation, the amount of the reaction solution is 2.1, the amount of the enzyme solution is 0.1, and Df is a dilution factor of the enzyme solution. The value "17.03" denotes the molecular weight of ammonia.

[Chemical Formula 1]

Enzymatic activity (U/mL)

= ammonia concentration in reaction solution (mg/L) × (1/17.03) × (amount of

reaction solution/amount of enzyme solution) × (1/10) × Df

(3) Confirmation of specific activity

The specific activity at 37°C was measured using the obtained enzyme samples of the modified protein glutaminases of Examples 1 to 3. The results showed that the specific activity when the specific activity of Chryseobacterium proteolyticum-derived protein glutaminase (wild-type) is defined as 100% was 83% for the enzyme sample of the modified protein glutaminase of Example 1, 109% for the enzyme sample of the modified protein glutaminase of Example 2, and 98% for the enzyme sample of the modified protein glutaminase of Example 3, and all the samples were confirmed to be equivalent in activity to the wild-type.

(4) Confirmation of ability to suppress decrease in reactivity at high temperature

The enzymatic activity at 60°C and 65°C was measured. The relative value of the enzymatic activity at a reaction temperature of 65°C when the enzymatic activity at a reaction temperature of 60°C is defined as 100% (relative activity $A_{65°C/60°C}$) was derived as an index of the degree of a decrease in reactivity at a high temperature. Further, the ratio of relative activity $A_{65°C/60°C}$ in the modified protein glutaminase of each of examples and comparative examples to relative activity $A_{65°C/60°C}$ in the wild-type protein glutaminase which is defined as 1 was derived as an index of suppression of a decrease in reactivity at a high temperature (vs. 60°C). The results are shown in the following table.

[Table 4]

|  |  | Relative activity $A_{65°C/60°C}$ | Index of suppression of decrease in reactivity at high temperature (vs. 60°C) |
|---|---|---|---|
| Reference Example 1 | Wild-type C. proteolyticum | 58% | 1 |
| Example 1 | P38A mutant | 65% | 1.11 |
| Example 2 | L156Y mutant | 74% | 1.28 |
| Example 3 | S72-T75 (RYGVKMSNQDG) mutant | 69% | 1.19 |
| Comparative Example 1 | K70R mutant | 60% | 1.03 |
| Comparative Example 2 | N119A mutant | 58% | 0.99 |

As shown in the table above, the index of suppression of a decrease in reactivity at a high temperature (vs. 60°C) was remarkably improved in the modified protein glutaminases of Examples 1 to 3.

(5) Confirmation of temperature stability

The residual activity (activity after heat treatment when the activity before heat treatment is defined as 100% (remaining percentage of activity)) of enzyme solutions made to contain equal amounts of protein and heat-treated at 50°C, 60°C, 65°C or 70°C for 10 minutes was measured. The results are shown in Fig. 1. After the treatment at 65°C for 10 minutes, the remaining activity of the wild-type protein glutaminase was 7%, whereas remaining activity of all the modified protein glutaminases of Example 1 to 3 was improved to 10% or more, and in particular, the remaining activity of the modified protein glutaminase of Example 2 was improved to 30% or more.

[Table 5]

|  |  | 50°C | 60°C | 65°C |
|---|---|---|---|---|
| Reference Example 1 | Wild-type C. proteolyticum | 98% | 77% | 7% |
| Example 1 | P38A mutant | 101% | 79% | 13% |
| Example 2 | L156Y mutant | 101% | 94% | 31% |
| Example 3 | S72-T75 (RYGVKMSNQDG) mutant | 102% | 85% | 14% |

Test Example 2

By the method for producing a P38A mutant in Test Example 1, a P38 saturated mutant was acquired using a primer designed to introduce a saturated mutation at position 38 in SEQ ID NO: 1. The following test was conducted on the modified protein glutaminases shown in Table 6, which were confirmed to have protein glutaminase activity, among the obtained mutants (other mutants had noticeably decreased activity).

For the modified protein glutaminases shown in Table 6, enzymatic activity at 57°C and 65°C was measured. The relative value of the enzymatic activity at a reaction temperature of 65°C when the enzymatic activity at a reaction temperature of 57°C is defined as 100% (relative activity $B_{65°C/57°C}$) was derived as an example of an index of the degree of a decrease in reactivity at a high temperature. Further, the ratio of relative activity $B_{65°C/57°C}$ in the modified protein glutaminase of each of examples and comparative examples to relative activity $B_{65°C/57°C}$ in the wild-type protein glutaminase which is defined as 1 was derived as an index of suppression of a decrease in reactivity at a high temperature (vs. 57°C). The results are shown in the following table.

[Table 6]

| | | Relative activity $B_{65°C/57°C}$ | Index of suppression of decrease in reactivity at high temperature (vs. 57°C) |
|---|---|---|---|
| Reference Example 1 | Wild-type C. proteolyticum | 60% | 1 |
| Example 1 | P38A mutant | 63% | 1.05 |
| Example 4 | P38F mutant | 108% | 1.80 |
| Example 5 | P38I mutant | 109% | 1.82 |
| Example 6 | P38L mutant | 145% | 2.42 |
| Example 7 | P38M mutant | 113% | 1.88 |
| Example 8 | P38R mutant | 66% | 1.10 |
| Example 9 | P38V mutant | 87% | 1.45 |
| Comparative Example 3 | P38W mutant | 41% | 0.68 |

As shown in the table above, the index of suppression of a decrease in reactivity at a high temperature (vs. 57°C) was confirmed to be improved in the modified protein glutaminases of Examples 1 and 4 to 9. From the results of the index of suppression of a decrease in reactivity based on the relative activity $A_{65°C/60°C}$ (vs. 60°C) for the modified protein glutaminase of Example 1 which are shown in Test Example 1, (4), it can be predicted that the protein glutaminases of Examples 4 to 9 also have a remarkably improved index of suppression of a decrease in reactivity (vs. 60°C).

Test Example 3

Using protein glutaminase derived from Chryseobacterium sp. (SEQ ID NO: 8), which is analogous protein glutaminase, instead of the amino acid sequence of protein glutaminase derived from C. proteolyticum (SEQ ID NO: 1), the P38 saturated mutant of SEQ ID NO: 8 was acquired in the same manner as in Test Example 2. SEQ ID NO: 8 has a sequence identity of 87.4% with SEQ ID NO: 1. For enzymes which were confirmed to have protein glutaminase activity, among the obtained mutants, the relative value of the enzymatic activity at a reaction temperature of 65°C (relative activity $A_{65°C/60°C}$) was derived as an index of the degree of a decrease in reactivity at a high temperature in the same manner as in Test Example 1. Further, the ratio of relative activity $A_{65°C/60°C}$ in the modified protein glutaminase of each of examples to relative activity $A_{65°C/60°C}$ in the wild-type protein glutaminase which is defined as 1 was derived as an index of suppression of a decrease in reactivity at a high temperature (vs. 60°C). The results are shown in the following table.

[Table 7]

| | | Relative activity $A_{65°C/60°C}$ | Index of suppression of decrease in reactivity at high temperature (vs. 60°C) |
|---|---|---|---|
| Reference Example 2 | Wild-type Chryseobacterium sp. | 96% | 1 |
| Example 10 | P38A mutant | 120% | 1.25 |
| Example 11 | P38F mutant | 128% | 1.33 |
| Example 12 | P38I mutant | 137% | 1.42 |
| Example 13 | P38L mutant | 152% | 1.58 |

(continued)

| | | Relative activity $A_{65°C/60°C}$ | Index of suppression of decrease in reactivity at high temperature (vs. 60°C) |
|---|---|---|---|
| Example 14 | P38M mutant | 149% | 1.55 |
| Example 15 | P38V mutant | 125% | 1.30 |
| Example 16 | P38Y mutant | 122% | 1.27 |
| Example 17 | P38C mutant | 114% | 1.18 |
| Example 18 | P38K mutant | 106% | 1.11 |
| Example 19 | P38S mutant | 111% | 1.16 |
| Example 20 | P38T mutant | 106% | 1.11 |

As shown in the table above, the index of suppression of a decrease in reactivity at a high temperature (vs. 60°C) was confirmed to be remarkably improved in the modified protein glutaminases of Examples 10 to 20.

Test Example 4

By the method for producing the mutant in Test Example 1, a F64 saturated mutant was acquired using a primer designed to introduce a saturated mutation at position 64 in SEQ ID NO: 1. The following test was conducted on the modified protein glutaminases shown in Table 8, which were confirmed to have protein glutaminase activity, among the obtained mutants (other mutants had noticeably decreased activity).

For the modified protein glutaminases shown in Table 8, enzymatic activity at 57°C and 65°C was measured. The relative value of the enzymatic activity at a reaction temperature of 65°C when the enzymatic activity at a reaction temperature of 57°C is defined as 100% (relative activity $B_{65°C/57°C}$) was derived as an example of an index of the degree of a decrease in reactivity at a high temperature. Further, the ratio of relative activity $B_{65°C/57°C}$ in the modified protein glutaminase of each of examples and comparative examples to relative activity $B_{65°C/57°C}$ in the wild-type protein glutaminase which is defined as 1 was derived as an index of suppression of a decrease in reactivity at a high temperature (vs. 57°C). The results are shown in the following table.

[Table 8]

| | | Index of suppression of decrease in reactivity at high temperature (vs. 57°C) |
|---|---|---|
| Reference Example 1 | Wild-type C. proteolyticum | 1 |
| Example 21 | F64E mutant | 1.17 |
| Comparative Example 5 | F64G mutant | 0.91 |
| Comparative Example 6 | F64V mutant | 0.79 |
| Comparative Example 7 | F64L mutant | 0.65 |

As shown in the table above, the index of suppression of a decrease in reactivity at a high temperature (vs. 57°C) was confirmed to be improved in the modified protein glutaminases of Example 21. From the results of the index of suppression of a decrease in reactivity based on the relative activity $A_{65°C/60°C}$ (vs. 60°C) for the modified protein glutaminase of Example 1 which are shown in Test Example 1, (4), it can be predicted that the protein glutaminase of Example 21 also has a remarkably improved index of suppression of a decrease in reactivity (vs. 60°C).

Test Example 5

By the method for producing the mutant in Test Example 1, a Y82 saturated mutant was acquired using a primer designed to introduce a saturated mutation at position 82 in SEQ ID NO: 1. The following test was conducted on the modified protein glutaminases shown in Table 9, which were confirmed to have protein glutaminase activity, among the obtained mutants (other mutants had noticeably decreased activity).

For the modified protein glutaminases shown in Table 9, enzymatic activity at 57°C and 65°C was measured. The relative value of the enzymatic activity at a reaction temperature of 65°C when the enzymatic activity at a reaction temperature of 57°C is defined as 100% (relative activity $B_{65°C/57°C}$) was derived as an example of an index of the degree of a decrease in reactivity at a high temperature. Further, the ratio of relative activity $B_{65°C/57°C}$ in the modified protein glutaminase of each of examples and comparative examples to relative activity $B_{65°C/57°C}$ in the wild-type protein glutaminase which is defined as 1 was derived as an index of suppression of a decrease in reactivity at a high temperature (vs. 57°C). The results are shown in the following table.

[Table 9]

|  |  | Index of suppression of decrease in reactivity at high temperature (vs. 57°C) |
| --- | --- | --- |
| Reference Example 1 | Wild-type C. proteolyticum | 1 |
| Example 22 | Y82P mutant | 2.01 |
| Example 23 | Y82W mutant | 1.83 |
| Example 24 | Y82F mutant | 1.78 |
| Comparative Example 8 | Y82C mutant | 0.83 |

As shown in the table above, the index of suppression of a decrease in reactivity at a high temperature (vs. 57°C) was confirmed to be improved in the modified protein glutaminases of Examples 22 to 24. From the results of the index of suppression of a decrease in reactivity based on the relative activity $A_{65°C/60°C}$ (vs. 60°C) for the modified protein glutaminase of Example 1 which are shown in Test Example 1, (4), it can be predicted that the protein glutaminases of Examples 22 to 24 also have a remarkably improved index of suppression of a decrease in reactivity (vs. 60°C).

Test Example 6

By the method for producing the mutant in Test Example 1, an A116 saturated mutant was acquired using a primer designed to introduce a saturated mutation at position 116 in SEQ ID NO: 1. The following test was conducted on the modified protein glutaminases shown in Table 10, which were confirmed to have protein glutaminase activity, among the obtained mutants (other mutants had noticeably decreased activity).

For the modified protein glutaminases shown in Table 10, enzymatic activity at 57°C and 65°C was measured. The relative value of the enzymatic activity at a reaction temperature of 65°C when the enzymatic activity at a reaction temperature of 57°C is defined as 100% (relative activity $B_{65°C/57°C}$) was derived as an example of an index of the degree of a decrease in reactivity at a high temperature. Further, the ratio of relative activity $B_{65°C/57°C}$ in the modified protein glutaminase of each of examples and comparative examples to relative activity $B_{65°C/57°C}$ in the wild-type protein glutaminase which is defined as 1 was derived as an index of suppression of a decrease in reactivity at a high temperature (vs. 57°C). The results are shown in the following table.

[Table 10]

|  |  | Index of suppression of decrease in reactivity at high temperature (vs. 57°C) |
| --- | --- | --- |
| Reference Example 1 | Wild-type C. proteolyticum | 1 |
| Example 25 | A116I mutant | 1.19 |
| Example 26 | A116V mutant | 1.14 |
| Example 27 | A116C mutant | 1.10 |
| Example 28 | A116L mutant | 1.08 |
| Example 29 | A116S mutant | 1.06 |
| Comparative Example 9 | A116G mutant | 0.95 |
| Comparative Example 10 | A116W mutant | 0.90 |
| Comparative Example 33 | A116F mutant | 0.83 |

As shown in the table above, the index of suppression of a decrease in reactivity at a high temperature (vs. 57°C) was confirmed to be improved in the modified protein glutaminases of Examples 25 to 29. From the results of the index of suppression of a decrease in reactivity based on the relative activity $A_{65°C/60°C}$ (vs. 60°C) for the modified protein glutaminase of Example 1 which are shown in Test Example 1, (4), it can be predicted that the protein glutaminases of Examples 25 to 29 also have a remarkably improved index of suppression of a decrease in reactivity (vs. 60°C).

Test Example 7

By the method for producing the mutant in Test Example 1, a N140 saturated mutant was acquired using a primer designed to introduce a saturated mutation at position 140 in SEQ ID NO: 1. The following test was conducted on the modified protein glutaminases shown in Table 11, which were confirmed to have protein glutaminase activity, among the obtained mutants (other mutants had noticeably decreased activity).

For the modified protein glutaminases shown in Table 11, enzymatic activity at 57°C and 65°C was measured. The relative value of the enzymatic activity at a reaction temperature of 65°C when the enzymatic activity at a reaction temperature of 57°C is defined as 100% (relative activity $B_{65°C/57°C}$) was derived as an example of an index of the degree of a decrease in reactivity at a high temperature. Further, the ratio of relative activity $B_{65°C/57°C}$ in the modified protein glutaminase of each of examples and comparative examples to relative activity $B_{65°C/57°C}$ in the wild-type protein glutaminase which is defined as 1 was derived as an index of suppression of a decrease in reactivity at a high temperature (vs. 57°C). The results are shown in the following table.

[Table 11]

| | | Index of suppression of decrease in reactivity at high temperature (vs. 57°C) |
|---|---|---|
| Reference Example 1 | Wild-type C. proteolyticum | 1 |
| Example 30 | A140I mutant | 1.41 |
| Example 31 | N140E mutant | 1.22 |
| Example 32 | N140Y mutant | 1.13 |
| Example 33 | N140D mutant | 1.11 |
| Example 34 | N140V mutant | 1.11 |
| Comparative Example 11 | N140W mutant | 0.98 |
| Comparative Example 12 | N140F mutant | 0.96 |
| Comparative Example 13 | N140R mutant | 0.92 |
| Comparative Example 14 | N140P mutant | 0.90 |
| Comparative Example 15 | N140L mutant | 0.84 |
| Comparative Example 16 | N140G mutant | 0.84 |

As shown in the table above, the index of suppression of a decrease in reactivity at a high temperature (vs. 57°C) was confirmed to be improved in the modified protein glutaminases of Examples 30 to 34. From the results of the index of suppression of a decrease in reactivity based on the relative activity $A_{65°C/60°C}$ (vs. 60°C) for the modified protein glutaminase of Example 1 which are shown in Test Example 1, (4), it can be predicted that the protein glutaminases of Examples 30 to 34 also have a remarkably improved index of suppression of a decrease in reactivity (vs. 60°C).

Test Example 8

By the method for producing the mutant in Test Example 1, an A2 saturated mutant was acquired using a primer designed to introduce a saturated mutation at position 2 in SEQ ID NO: 1. The following test was conducted on the modified protein glutaminases shown in Table 12, which were confirmed to have protein glutaminase activity, among the obtained mutants (other mutants had noticeably decreased activity).

For the modified protein glutaminases shown in Table 12, enzymatic activity at 57°C and 65°C was measured. The relative value of the enzymatic activity at a reaction temperature of 65°C when the enzymatic activity at a reaction temperature of 57°C is defined as 100% (relative activity $B_{65°C/57°C}$) was derived as an example of an index of the degree of a decrease in reactivity at a high temperature. Further, the ratio of relative activity $B_{65°C/57°C}$ in the modified protein glutaminase of each of examples to relative activity $B_{65°C/57°C}$ in the wild-type protein glutaminase which is defined as 1 was derived as an index of suppression of a decrease in reactivity at a high temperature (vs. 57°C). The results are shown in the following table.

[Table 12]

| | | Index of suppression of decrease in reactivity at high temperature (vs. 57°C) |
|---|---|---|
| Reference Example 1 | Wild-type C. proteolyticum | 1 |
| Example 35 | A2R mutant | 1.08 |
| Example 36 | A2W mutant | 1.37 |

As shown in the table above, the index of suppression of a decrease in reactivity at a high temperature (vs. 57°C) was confirmed to be improved in the modified protein glutaminases of Examples 35 and 36. From the results of the index of suppression of a decrease in reactivity based on the relative activity $A_{65°C/60°C}$ (vs. 60°C) for the modified protein glutaminase of Example 1 which are shown in Test Example 1, (4), it can be predicted that the protein glutaminases of Examples 35 and 36 also has a remarkably improved index of suppression of a decrease in reactivity (vs. 60°C).

Test Example 9

By the method for producing the mutant in Test Example 1, a S3 saturated mutant was acquired using a primer designed to introduce a saturated mutation at position 3 in SEQ ID NO: 1. The following test was conducted on the modified protein glutaminases shown in Table 13, which were confirmed to have protein glutaminase activity, among the obtained mutants (other mutants had noticeably decreased activity).

For the modified protein glutaminases shown in Table 13, enzymatic activity at 57°C and 65°C was measured. The relative value of the enzymatic activity at a reaction temperature of 65°C when the enzymatic activity at a reaction temperature of 57°C is defined as 100% (relative activity $B_{65°C/57°C}$) was derived as an example of an index of the degree of a decrease in reactivity at a high temperature. Further, the ratio of relative activity $B_{65°C/57°C}$ in the modified protein glutaminase of examples to relative activity $B_{65°C/57°C}$ in the wild-type protein glutaminase which is defined as 1 was derived as an index of suppression of a decrease in reactivity at a high temperature (vs. 57°C). The results are shown in the following table.

[Table 13]

| | | Index of suppression of decrease in reactivity at high temperature (vs. 57°C) |
|---|---|---|
| Reference Example 1 | Wild-type C. proteolyticum | 1 |
| Example 37 | S3R mutant | 1.37 |

As shown in the table above, the index of suppression of a decrease in reactivity at a high temperature (vs. 57°C) was confirmed to be improved in the modified protein glutaminases of Example 37. From the results of the index of suppression of a decrease in reactivity based on the relative activity $A_{65°C/60°C}$ (vs. 60°C) for the modified protein glutaminase of Example 1 which are shown in Test Example 1, (4), it can be predicted that the protein glutaminase of Example 37 also has a remarkably improved index of suppression of a decrease in reactivity (vs. 60°C).

Test Example 10

By the method for producing the mutant in Test Example 1, a N16 saturated mutant was acquired using a primer designed to introduce a saturated mutation at position 16 in SEQ ID NO: 1. The following test was conducted on the modified protein glutaminases shown in Table 14, which were confirmed to have protein glutaminase activity, among the obtained mutants (other mutants had noticeably decreased activity).

For the modified protein glutaminases shown in Table 14, enzymatic activity at 57°C and 65°C was measured. The relative value of the enzymatic activity at a reaction temperature of 65°C when the enzymatic activity at a reaction temperature of 57°C is defined as 100% (relative activity $B_{65°C/57°C}$) was derived as an example of an index of the degree of a decrease in reactivity at a high temperature. Further, the ratio of relative activity $B_{65°C/57°C}$ in the modified protein glutaminase of each of examples and comparative examples to relative activity $B_{65°C/57°C}$ in the wild-type protein glutaminase which is defined as 1 was derived as an index of suppression of a decrease in reactivity at a high temperature (vs. 57°C). The results are shown in the following table.

[Table 14]

| | | Index of suppression of decrease in reactivity at high temperature (vs. 57°C) |
|---|---|---|
| Reference Example 1 | Wild-type C. proteolyticum | 1 |
| Example 38 | N16R mutant | 1.12 |
| Example 39 | N16F mutant | 1.14 |
| Example 40 | N16W mutant | 1.23 |
| Example 41 | N16S mutant | 1.23 |
| Example 42 | N16A mutant | 1.32 |
| Example 43 | N16M mutant | 1.42 |
| Example 44 | N16C mutant | 1.45 |
| Example 45 | N16V mutant | 1.46 |
| Example 46 | N16L mutant | 1.5 |
| Comparative Example 17 | N16G mutant | 0.96 |

As shown in the table above, the index of suppression of a decrease in reactivity at a high temperature (vs. 57°C) was confirmed to be improved in the modified protein glutaminases of Examples 38 and 46. From the results of the index of suppression of a decrease in reactivity based on the relative activity $A_{65°C/60°C}$ (vs. 60°C) for the modified protein glutaminase of Example 1 which are shown in Test Example 1, (4), it can be predicted that the protein glutaminases of Examples 38 and 46 also has a remarkably improved index of suppression of a decrease in reactivity (vs. 60°C).

Test Example 11

By the method for producing the mutant in Test Example 1, an I86 saturated mutant was acquired using a primer designed to introduce a saturated mutation at position 86 in SEQ ID NO: 1. The following test was conducted on the modified protein glutaminases shown in Table 15, which were confirmed to have protein glutaminase activity, among the obtained mutants (other mutants had noticeably decreased activity).

For the modified protein glutaminases shown in Table 15, enzymatic activity at 57°C and 65°C was measured. The relative value of the enzymatic activity at a reaction temperature of 65°C when the enzymatic activity at a reaction temperature of 57°C is defined as 100% (relative activity $B_{65°C/57°C}$) was derived as an example of an index of the degree of a decrease in reactivity at a high temperature. Further, the ratio of relative activity $B_{65°C/57°C}$ in the modified protein glutaminase of examples to relative activity $B_{65°C/57°C}$ in the wild-type protein glutaminase which is defined as 1 was derived as an index of suppression of a decrease in reactivity at a high temperature (vs. 57°C). The results are shown in the following table.

[Table 15]

| | | Index of suppression of decrease in reactivity at high temperature (vs. 57°C) |
|---|---|---|
| Reference Example 1 | Wild-type C. proteolyticum | 1 |
| Example 47 | I86P mutant | 1.36 |

As shown in the table above, the index of suppression of a decrease in reactivity at a high temperature (vs. 57°C) was confirmed to be improved in the modified protein glutaminases of Example 47. From the results of the index of suppression of a decrease in reactivity based on the relative activity $A_{65°C/60°C}$ (vs. 60°C) for the modified protein glutaminase of Example 1 which are shown in Test Example 1, (4), it can be predicted that the protein glutaminase of Example 47 also has a remarkably improved index of suppression of a decrease in reactivity (vs. 60°C).

Test Example 12

By the method for producing the mutant in Test Example 1, a saturated mutant at each amino acid site was acquired using a primer designed to introduce a saturated mutation at position 5, 9, 11, 13, 17, 19, 20, 22, 26, 30, 50, 51, 52, 53, 54, 57 or 58 in SEQ ID NO: 1. The following test was conducted on the modified protein glutaminases shown in Tables 16 to 32, which were confirmed to have protein glutaminase activity, among the obtained mutants (other mutants had noticeably decreased activity).

For the modified protein glutaminases shown in Tables 16 to 32, enzymatic activity at 57°C and 65°C was measured. The relative value of the enzymatic activity at a reaction temperature of 65°C when the enzymatic activity at a reaction temperature of 57°C is defined as 100% (relative activity $B_{65°C/57°C}$) was derived as an example of an index of the degree of a decrease in reactivity at a high temperature. Further, the ratio of relative activity $B_{65°C/57°C}$ in the modified protein glutaminase of each of examples and comparative examples to relative activity $B_{65°C/57°C}$ in the wild-type protein glutaminase which is defined as 1 was derived as an index of suppression of a decrease in reactivity at a high temperature (vs. 57°C). The results are shown in the following table.

[Table 16]

| | | Index of suppression of decrease in reactivity at high temperature (vs. 57°C) |
|---|---|---|
| Reference Example 1 | Wild-type C. proteolyticum | 1 |
| Example 48 | I5V mutant | 1.14 |
| Example 49 | I5L mutant | 1.28 |
| Example 50 | I5T mutant | 1.34 |
| Comparative Example 18 | I5A mutant | 0.89 |

[Table 17]

| | | Index of suppression of decrease in reactivity at high temperature (vs. 57°C) |
|---|---|---|
| Reference Example 1 | Wild-type C. proteolyticum | 1 |
| Example 51 | A9E mutant | 1.06 |
| Example 52 | A9D mutant | 1.08 |
| Example 53 | A9M mutant | 1.09 |
| Example 54 | A9G mutant | 1.13 |
| Example 55 | A9W mutant | 1.15 |
| Example 56 | A9R mutant | 1.17 |
| Example 57 | A9C mutant | 1.19 |
| Example 58 | A9P mutant | 1.27 |
| Example 59 | A9F mutant | 1.27 |
| Comparative Example 19 | A9L mutant | 0.95 |

[Table 18]

| | | Index of suppression of decrease in reactivity at high temperature (vs. 57°C) |
|---|---|---|
| Reference Example 1 | Wild-type C. proteolyticum | 1 |
| Example 60 | L11H mutant | 1.14 |
| Example 61 | L11M mutant | 1.20 |
| Example 62 | L11V mutant | 1.24 |
| Example 63 | L11G mutant | 1.32 |

(continued)

|  |  | Index of suppression of decrease in reactivity at high temperature (vs. 57°C) |
| --- | --- | --- |
| Example 64 | L11C mutant | 1.34 |
| Example 65 | L11R mutant | 1.44 |

[Table 19]

|  |  | Index of suppression of decrease in reactivity at high temperature (vs. 57°C) |
| --- | --- | --- |
| Reference Example 1 | Wild-type C. proteolyticum | 1 |
| Example 66 | S13M mutant | 1.05 |
| Example 67 | S13N mutant | 1.07 |
| Example 68 | S13W mutant | 1.27 |
| Example 69 | S13F mutant | 1.29 |
| Comparative Example 20 | S13T mutant | 0.91 |
| Comparative Example 21 | S13Y mutant | 0.92 |

[Table 20]

|  |  | Index of suppression of decrease in reactivity at high temperature (vs. 57°C) |
| --- | --- | --- |
| Reference Example 1 | Wild-type C. proteolyticum | 1 |
| Example 70 | Q17G mutant | 1.10 |
| Example 71 | Q17A mutant | 1.22 |
| Comparative Example 22 | Q17C mutant | 0.95 |

[Table 21]

|  |  | Index of suppression of decrease in reactivity at high temperature (vs. 57°C) |
| --- | --- | --- |
| Reference Example 1 | Wild-type C. proteolyticum | 1 |
| Example 72 | K19L mutant | 1.15 |
| Example 73 | K19T mutant | 1.21 |
| Example 74 | K19S mutant | 1.24 |
| Example 75 | K19M mutant | 1.28 |
| Example 76 | K19R mutant | 1.39 |

[Table 22]

|  |  | Index of suppression of decrease in reactivity at high temperature (vs. 57°C) |
| --- | --- | --- |
| Reference Example 1 | Wild-type C. proteolyticum | 1 |
| Example 77 | N20G mutant | 1.06 |
| Example 78 | N20E mutant | 1.17 |
| Example 79 | N20S mutant | 1.23 |
| Comparative Example 23 | N20A mutant | 0.80 |

(continued)

|  | | Index of suppression of decrease in reactivity at high temperature (vs. 57°C) |
|---|---|---|
| Comparative Example 24 | N20L mutant | 0.88 |
| Comparative Example 25 | N201 mutant | 0.91 |

[Table 23]

|  | | Index of suppression of decrease in reactivity at high temperature (vs. 57°C) |
|---|---|---|
| Reference Example 1 | Wild-type C. proteolyticum | 1 |
| Example 80 | S22G mutant | 1.34 |

[Table 24]

|  | | Index of suppression of decrease in reactivity at high temperature (vs. 57°C) |
|---|---|---|
| Reference Example 1 | Wild-type C. proteolyticum | 1 |
| Example 81 | S26V mutant | 1.18 |

[Table 25]

|  | | Index of suppression of decrease in reactivity at high temperature (vs. 57°C) |
|---|---|---|
| Reference Example 1 | Wild-type C. proteolyticum | 1 |
| Example 82 | S30Y mutant | 1.12 |
| Example 83 | S30C mutant | 1.19 |
| Example 84 | S30V mutant | 1.2 |
| Example 85 | S30H mutant | 1.26 |
| Example 87 | S30R mutant | 1.41 |
| Example 88 | S30F mutant | 1.44 |
| Example 136 | S30Q mutant | 1.19 |
| Comparative Example 26 | S30L mutant | 0.99 |

[Table 26]

|  | | Index of suppression of decrease in reactivity at high temperature (vs. 57°C) |
|---|---|---|
| Reference Example 1 | Wild-type C. proteolyticum | 1 |
| Example 89 | R50W mutant | 1.48 |

[Table 27]

|  | | Index of suppression of decrease in reactivity at high temperature (vs. 57°C) |
|---|---|---|
| Reference Example 1 | Wild-type C. proteolyticum | 1 |
| Example 90 | Q51P mutant | 1.09 |
| Example 91 | Q51H mutant | 1.09 |
| Example 92 | Q51W mutant | 1.55 |

(continued)

|  |  | Index of suppression of decrease in reactivity at high temperature (vs. 57°C) |
| --- | --- | --- |
| Example 137 | Q51F mutant | 1.06 |
| Comparative Example 27 | Q51G mutant | 0.75 |
| Comparative Example 31 | Q51S mutant | 0.92 |
| Comparative Example 32 | Q51A mutant | 0.99 |

[Table 28]

|  |  | Index of suppression of decrease in reactivity at high temperature (vs. 57°C) |
| --- | --- | --- |
| Reference Example 1 | Wild-type C. proteolyticum | 1 |
| Example 93 | I52V mutant | 1.20 |

[Table 29]

|  |  | Index of suppression of decrease in reactivity at high temperature (vs. 57°C) |
| --- | --- | --- |
| Reference Example 1 | Wild-type C. proteolyticum | 1 |
| Example 94 | L53Q mutant | 1.25 |
| Comparative Example 28 | L53K mutant | 0.98 |
| Comparative Example 29 | L53V mutant | 0.93 |

[Table 30]

|  |  | Index of suppression of decrease in reactivity at high temperature (vs. 57°C) |
| --- | --- | --- |
| Reference Example 1 | Wild-type C. proteolyticum | 1 |
| Example 95 | M54N mutant | 1.22 |
| Comparative Example 30 | M54V mutant | 0.83 |

[Table 31]

|  |  | Index of suppression of decrease in reactivity at high temperature (vs. 57°C) |
| --- | --- | --- |
| Reference Example 1 | Wild-type C. proteolyticum | 1 |
| Example 96 | G57C mutant | 1.11 |

[Table 32]

|  |  | Index of suppression of decrease in reactivity at high temperature (vs. 57°C) |
| --- | --- | --- |
| Reference Example 1 | Wild-type C. proteolyticum | 1 |
| Example 97 | Y58T mutant | 1.28 |

As shown in the tables above, the index of suppression of a decrease in reactivity at a high temperature (vs. 57°C) was confirmed to be improved in the modified protein glutaminases of Examples 48 to 88, 136, 89 to 92, 137 and 93 to 97. From the results of the index of suppression of a decrease in reactivity based on the relative activity $A_{65°C/60°C}$ (vs. 60°C) for the modified protein glutaminase of Example 1 which are shown in Test Example 1, (4), it can be predicted that the protein glutaminases of Examples 48 to 88, 136, 89 to 92, 137 and 93 to 97 also have a remarkably improved index of suppression of a decrease in reactivity (vs. 60°C).

Test Example 13

By the method for producing a mutant in Test Example 3, a mutant at each amino acid site was acquired using protein glutaminase derived from Chryseobacterium sp. (SEQ ID NO: 8), which is analogous protein glutaminase, and a primer designed to introduce the mutation shown in Table 33 into at position 73, 73 to 75, or 79 in SEQ ID NO: 8. The following test was conducted on the modified protein glutaminases shown in Table 33, which were confirmed to have protein glutaminase activity, among the obtained mutants.

For the modified protein glutaminases shown in Table 33, enzymatic activity at 60°C and 65°C was measured. The relative value of the enzymatic activity at a reaction temperature of 65°C when the enzymatic activity at a reaction temperature of 60°C is defined as 100% (relative activity $A_{65°C/60°C}$) was derived as an index of the degree of a decrease in reactivity at a high temperature. Further, the ratio of relative activity $A_{65°C/60°C}$ in the modified protein glutaminase of each of examples to relative activity $A_{65°C/60°C}$ in the wild-type protein glutaminase which is defined as 1 was derived as an index of suppression of a decrease in reactivity at a high temperature (vs. 60°C). The results are shown in the following table.

[Table 33]

| | | Index of suppression of decrease in reactivity at high temperature (vs. 60°C) |
|---|---|---|
| Reference Example 2 | Wild-type Chryseobacterium sp. | 1 |
| Example 99 | T73V mutant | 1.18 |
| Example 100 | T73P/G74M/T75G mutant | 1.13 |
| Example 101 | S79K mutant | 1.23 |
| Example 102 | S79R mutant | 1.06 |

As shown in the table above, the index of suppression of a decrease in reactivity at a high temperature (vs. 60°C) was confirmed to be improved in the modified protein glutaminases of Examples 99 and 102.

Test Example 14

By the method for producing the mutant in Test Example 1, a saturated mutant at each amino acid site was acquired using a primer designed to introduce a saturated mutation at position 92, 94, 95, 96, 97, 160 or 164 in SEQ ID NO: 1. The following test was conducted on the modified protein glutaminases shown in Tables 34 to 40, which were confirmed to have protein glutaminase activity, among the obtained mutants (other mutants had noticeably decreased activity).

For the modified protein glutaminases shown in Tables 34 to 40, enzymatic activity at 57°C and 65°C was measured. The relative value of the enzymatic activity at a reaction temperature of 65°C when the enzymatic activity at a reaction temperature of 57°C is defined as 100% (relative activity $B_{65°C/57°C}$) was derived as an example of an index of the degree of a decrease in reactivity at a high temperature. Further, the ratio of relative activity $B_{65°C/57°C}$ in the modified protein glutaminase of each of examples to relative activity $B_{65°C/57°C}$ in the wild-type protein glutaminase which is defined as 1 was derived as an index of suppression of a decrease in reactivity at a high temperature (vs. 57°C). The results are shown in the following table.

[Table 34]

| | | Index of suppression of decrease in reactivity at high temperature (vs. 57°C) |
|---|---|---|
| Reference Example 1 | Wild-type C. proteolyticum | 1 |
| Example 104 | N92S mutant | 1.09 |
| Example 105 | M92V mutant | 1.15 |
| Example 106 | M92G mutant | 1.19 |

[Table 35]

| | | Index of suppression of decrease in reactivity at high temperature (vs. 57°C) |
|---|---|---|
| Reference Example 1 | Wild-type C. proteolyticum | 1 |
| Example 107 | S94Y mutant | 1.09 |
| Example 108 | S94W mutant | 1.20 |

[Table 36]

| | | Index of suppression of decrease in reactivity at high temperature (vs. 57°C) |
|---|---|---|
| Reference Example 1 | Wild-type C. proteolyticum | 1 |
| Example 109 | G95F mutant | 1.24 |
| Example 110 | G95D mutant | 1.48 |
| Example 138 | G95C mutant | 1.18 |
| Example 139 | G95M mutant | 1.35 |

[Table 37]

| | | Index of suppression of decrease in reactivity at high temperature (vs. 57°C) |
|---|---|---|
| Reference Example 1 | Wild-type C. proteolyticum | 1 |
| Example 111 | V96K mutant | 1.06 |
| Example 112 | V96Q mutant | 1.07 |
| Example 113 | V96F mutant | 1.11 |
| Example 114 | V96N mutant | 1.12 |
| Example 115 | V96Y mutant | 1.16 |
| Example 116 | V96L mutant | 1.17 |
| Example 117 | V96R mutant | 1.20 |
| Example 118 | V96W mutant | 1.20 |
| Example 119 | V96I mutant | 1.28 |
| Example 120 | V96G mutant | 1.30 |
| Example 121 | V96S mutant | 1.34 |
| Example 122 | V96T mutant | 1.37 |
| Example 123 | V96A mutant | 1.37 |
| Example 140 | V96D mutant | 1.06 |
| Example 141 | V96C mutant | 1.07 |
| Example 142 | V96M mutant | 1.09 |

[Table 38]

| | | Index of suppression of decrease in reactivity at high temperature (vs. 57°C) |
|---|---|---|
| Reference Example 1 | Wild-type C. proteolyticum | 1 |
| Example 124 | T97K mutant | 1.09 |

[Table 39]

| | | Index of suppression of decrease in reactivity at high temperature (vs. 57°C) |
|---|---|---|
| Reference Example 1 | Wild-type C. proteolyticum | 1 |
| Example 125 | N160F mutant | 1.08 |
| Example 126 | N160D mutant | 1.27 |

[Table 40]

| | | Index of suppression of decrease in reactivity at high temperature (vs. 57°C) |
|---|---|---|
| Reference Example 1 | Wild-type C. proteolyticum | 1 |
| Example 127 | T164D mutant | 1.06 |
| Example 128 | T164H mutant | 1.06 |
| Example 129 | T164F mutant | 1.07 |
| Example 130 | T164S mutant | 1.19 |
| Example 131 | T164A mutant | 1.21 |
| Example 132 | T164C mutant | 1.22 |
| Example 133 | T164L mutant | 1.22 |
| Example 134 | T164M mutant | 1.37 |
| Example 135 | T164R mutant | 1.43 |

As shown in the tables above, the index of suppression of a decrease in reactivity at a high temperature (vs. 57°C) was confirmed to be improved in the modified protein glutaminases of Examples 104 to 110, 138, 139, 111 to 123, 140 to 142 and 124 to 135. From the results of the index of suppression of a decrease in reactivity based on the relative activity $A_{65°C/60°C}$ (vs. 60°C) for the modified protein glutaminase of Example 1 which are shown in Test Example 1, (4), it can be predicted that the protein glutaminases of Examples 104 to 110, 138, 139, 111 to 123, 140 to 142, 124 to 135 also have a remarkably improved index of suppression of a decrease in reactivity (vs. 60°C).

Test Example 15

By the method for producing the mutant in Test Example 1, a saturated mutant at each amino acid site was acquired using a primer designed to introduce a saturated mutation at position 12, 24, 98, 131, 134, 135, 136, 168, 175, 182 or 184 in SEQ ID NO: 1. The following test was conducted on the modified protein glutaminases shown in Tables 41 to 51, which were confirmed to have protein glutaminase activity, among the obtained mutants (other mutants had noticeably decreased activity).

For the modified protein glutaminases shown in Tables 41 to 51, enzymatic activity at 57°C and 65°C was measured. The relative value of the enzymatic activity at a reaction temperature of 65°C when the enzymatic activity at a reaction temperature of 57°C is defined as 100% (relative activity $B_{65°C/57°C}$) was derived as an example of an index of the degree of a decrease in reactivity at a high temperature. Further, the ratio of relative activity $B_{65°C/57°C}$ in the modified protein glutaminase of each of examples to relative activity $B_{65°C/57°C}$ in the wild-type protein glutaminase which is defined as 1 was derived as an index of suppression of a decrease in reactivity at a high temperature (vs. 57°C). The results are shown in the following table.

[Table 41]

| | | Index of suppression of decrease in reactivity at high temperature (vs. 57°C) |
|---|---|---|
| Reference Example 1 | Wild-type C. proteolyticum | 1 |
| Example 143 | N12Y mutant | 1.13 |
| Example 144 | N12M mutant | 1.15 |

(continued)

| | | Index of suppression of decrease in reactivity at high temperature (vs. 57°C) |
|---|---|---|
| Example 145 | N12W mutant | 1.15 |
| Example 146 | N12C mutant | 1.20 |

[Table 42]

| | | Index of suppression of decrease in reactivity at high temperature (vs. 57°C) |
|---|---|---|
| Reference Example 1 | Wild-type C. proteolyticum | 1 |
| Example 147 | G24S mutant | 1.19 |

[Table 43]

| | | Index of suppression of decrease in reactivity at high temperature (vs. 57°C) |
|---|---|---|
| Reference Example 1 | Wild-type C. proteolyticum | 1 |
| Example 148 | E98F mutant | 1.27 |

[Table 44]

| | | Index of suppression of decrease in reactivity at high temperature (vs. 57°C) |
|---|---|---|
| Reference Example 1 | Wild-type C. proteolyticum | 1 |
| Example 149 | V131L mutant | 1.14 |

[Table 45]

| | | Index of suppression of decrease in reactivity at high temperature (vs. 57°C) |
|---|---|---|
| Reference Example 1 | Wild-type C. proteolyticum | 1 |
| Example 150 | Y134W mutant | 1.20 |

[Table 46]

| | | Index of suppression of decrease in reactivity at high temperature (vs. 57°C) |
|---|---|---|
| Reference Example 1 | Wild-type C. proteolyticum | 1 |
| Example 151 | A135I mutant | 1.05 |
| Example 152 | A135C mutant | 1.09 |
| Example 153 | A135F mutant | 1.23 |
| Example 154 | A135V mutant | 1.39 |

[Table 47]

| | | Index of suppression of decrease in reactivity at high temperature (vs. 57°C) |
|---|---|---|
| Reference Example 1 | Wild-type C. proteolyticum | 1 |
| Example 155 | N1361 mutant | 1.06 |

(continued)

|  |  | Index of suppression of decrease in reactivity at high temperature (vs. 57°C) |
|---|---|---|
| Example 156 | N136R mutant | 1.07 |
| Example 157 | N136L mutant | 1.08 |
| Example 158 | N136V mutant | 1.23 |

[Table 48]

|  |  | Index of suppression of decrease in reactivity at high temperature (vs. 57°C) |
|---|---|---|
| Reference Example 1 | Wild-type C. proteolyticum | 1 |
| Example 159 | L168R mutant | 1.09 |
| Example 173 | L168N mutant | 1.15 |
| Example 174 | L168C mutant | 1.16 |

[Table 49]

|  |  | Index of suppression of decrease in reactivity at high temperature (vs. 57°C) |
|---|---|---|
| Reference Example 1 | Wild-type C. proteolyticum | 1 |
| Example 160 | S175T mutant | 1.13 |
| Example 161 | S175A mutant | 1.14 |
| Example 162 | S175V mutant | 1.18 |
| Example 163 | S175C mutant | 1.28 |

[Table 50]

|  |  | Index of suppression of decrease in reactivity at high temperature (vs. 57°C) |
|---|---|---|
| Reference Example 1 | Wild-type C. proteolyticum | 1 |
| Example 164 | S182L mutant | 1.08 |
| Example 165 | S182M mutant | 1.13 |
| Example 166 | S182E mutant | 1.14 |
| Example 167 | S182Q mutant | 1.15 |
| Example 168 | S182G mutant | 1.21 |
| Example 169 | S182D mutant | 1.27 |
| Example 170 | S182A mutant | 1.30 |

[Table 51]

|  |  | Index of suppression of decrease in reactivity at high temperature (vs. 57°C) |
|---|---|---|
| Reference Example 1 | Wild-type C. proteolyticum | 1 |
| Example 171 | G184W mutant | 1.21 |
| Example 172 | G184F mutant | 1.58 |

As shown in the table above, the index of suppression of a decrease in reactivity at a high temperature (vs. 57°C) was confirmed to be improved in the modified protein glutaminases of Examples 143 and 174. From the results of the index of suppression of a decrease in reactivity based on the relative activity $A_{65°C/60°C}$ (vs. 60°C) for the modified protein glutaminase of Example 1 which are shown in Test Example 1, (4), it can be predicted that the protein glutaminases of Examples 143 to 174 also have a remarkably improved index of suppression of a decrease in reactivity (vs. 60°C).

Sequence Listing Free Text

SEQ ID NO: 2 is a sequence newly found through the present invention to impart the property of suppressing a decrease in reactivity at a high temperature to protein glutaminase.
SEQ ID NOS: 18 to 23 correspond to primers.

**Claims**

1. A modified protein glutaminase comprising one of the following polypeptides (1) to (III):

(I) a polypeptide consisting of an amino acid sequence obtained by introducing at least one of

(A) a substitution of the amino acid residue at position 38 with an alanine residue, a phenylalanine residue, an isoleucine residue, a leucine residue, a methionine residue, an arginine residue, a valine residue, a tyrosine residue, a cysteine residue, a lysine residue, a serine residue, or a threonine residue,
(B) a substitution of the amino acid residue at position 156 with a tyrosine residue,
(C) a substitution of the four consecutive amino acid residues at positions 72 to 75 with an amino acid sequence consisting of RYGVKMSNQDG (SEQ ID NO: 2),
(D) a substitution of the amino acid residue at position 64 with a glutamic acid residue,
(E) a substitution of the amino acid residue at position 82 with a proline residue, a tryptophan residue, or a phenylalanine residue,
(F) a substitution of the amino acid residue at position 116 with an isoleucine residue, a valine residue, a cysteine residue, a leucine residue, or a serine residue,
(G) a substitution of the amino acid residue at position 140 with an isoleucine residue, a glutamic acid residue, a tyrosine residue, an aspartic acid residue, or a valine residue,
(H) a substitution of the amino acid residue at position 2 with an arginine residue or a tryptophan residue,
(I) a substitution of the amino acid residue at position 3 with an arginine residue,
(J) a substitution of the amino acid residue at position 16 with an alanine residue, a cysteine residue, a phenylalanine residue, a leucine residue, a methionine residue, an arginine residue, a serine residue, a valine residue, or a tryptophan residue,
(K) a substitution of the amino acid residue at position 86 with a proline residue,
(L) a substitution of the amino acid residue at position 5 with a leucine residue, a threonine residue, or a valine residue,
(M) a substitution of the amino acid residue at position 9 with a glutamic acid residue, an aspartic acid residue, a methionine residue, a cysteine residue, a phenylalanine residue, a glycine residue, a proline residue, an arginine residue, or a tryptophan residue,
(N) a substitution of the amino acid residue at position 11 with a cysteine residue, a glycine residue, a histidine residue, a methionine residue, an arginine residue, or a valine residue,
(O) a substitution of the amino acid residue at position 13 with a methionine residue, an asparagine residue, a phenylalanine residue, or a tryptophan residue,
(P) a substitution of the amino acid residue at position 17 with a glycine residue or an alanine residue,
(Q) a substitution of the amino acid residue at position 19 with a leucine residue, a methionine residue, an arginine residue, a serine residue, or a threonine residue,
(R) a substitution of the amino acid residue at position 20 with a glycine residue, a glutamic acid residue, or a serine residue,
(S) a substitution of the amino acid residue at position 22 with a glycine residue,
(T) a substitution of the amino acid residue at position 26 with a valine residue,
(U) a substitution of the amino acid residue at position 30 with a cysteine residue, a phenylalanine residue, a histidine residue, an arginine residue, a valine residue, a tyrosine residue, or a glutamine residue,
(V) a substitution of the amino acid residue at position 50 with a tryptophan residue,
(W) a substitution of the amino acid residue at position 51 with a proline residue, a histidine residue, a tryptophan residue, or a phenylalanine residue,

(X) a substitution of the amino acid residue at position 52 with a valine residue,

(Y) a substitution of the amino acid residue at position 53 with a glutamine residue,

(Z) a substitution of the amino acid residue at position 54 with an asparagine residue,

(AA) a substitution of the amino acid residue at position 57 with a cysteine residue,

(AB) a substitution of the amino acid residue at position 58 with a threonine residue,

(AD) a substitution of the amino acid residue at position 73 with a valine residue,

(AE) a substitution of the three consecutive amino acid residues at positions 73 to 75 with an amino acid sequence consisting of a PMG,

(AF) a substitution of the amino acid residue at position 79 with a lysine residue or an arginine residue,

(AR) a substitution of the amino acid residue at position 92 with a glycine residue, a serine residue, or a valine residue,

(AI) a substitution of the amino acid residue at position 94 with a tryptophan residue or a tyrosine residue,

(AJ) a substitution of the amino acid residue at position 95 with an aspartic acid residue, a phenylalanine residue, a cysteine residue, or a methionine residue,

(AK) a substitution of the amino acid residue at position 96 with an alanine residue, a phenylalanine residue, a glycine residue, an isoleucine residue, a lysine residue, a leucine residue, an asparagine residue, a glutamine residue, an arginine residue, a serine residue, a threonine residue, a tryptophan residue, a tyrosine residue, an aspartic acid residue, a cysteine residue, or a methionine residue,

(AL) a substitution of the amino acid residue at position 97 with a lysine residue,

(AM) a substitution of the amino acid residue at position 160 with an aspartic acid residue or a phenylalanine residue,

(AN) a substitution of the amino acid residue at position 164 with an alanine residue, a cysteine residue, an aspartic acid residue, a phenylalanine residue, a histidine residue, a leucine residue, a methionine residue, an arginine residue, or a serine residue,

(AO) a substitution of the amino acid residue at position 12 with a cysteine residue, a methionine residue, a tryptophan residue, or a tyrosine residue,

(AP) a substitution of the amino acid residue at position 24 with a serine residue,

(AQ) a substitution of the amino acid residue at position 98 with a phenylalanine residue,

(AR) a substitution of the amino acid residue at position 131 with a leucine residue,

(AS) a substitution of the amino acid residue at position 134 with a tryptophan residue,

(AT) a substitution of the amino acid residue at position 135 with an isoleucine residue, a cysteine residue, a phenylalanine residue, or a valine residue,

(AU) a substitution of the amino acid residue at position 136 with an isoleucine residue, an arginine residue, a leucine residue, or a valine residue,

(AV) a substitution of the amino acid residue at position 168 with an arginine residue, an asparagine residue, or a cysteine residue,

(AW) a substitution of the amino acid residue at position 175 with a threonine residue, an alanine residue, a valine residue, or a cysteine residue,

(AX) a substitution of the amino acid residue at position 182 with a leucine residue, a methionine residue, a glutamic acid residue, a glutamine residue, a glycine residue, an aspartic acid residue or an alanine residue, and

(AY) a substitution of the amino acid residue at position 184 with a tryptophan residue or a phenylalanine residue,

into the amino acid sequence set forth as SEQ ID NO: 1;

(II) a polypeptide in which one or several amino acid residues other than the substituted amino acid residues are substituted, added, inserted or deleted in the amino acid sequence in which at least one of the substitutions (A) to (AY) is introduced, and the relative activity of protein glutaminase at 65°C with respect to the activity of protein glutaminase at 60°C is improved as compared to the relative activity of the polypeptide consisting of the amino acid sequence set forth as SEQ ID NO: 1; and

(III) a polypeptide in which the sequence identity of regions that do not include the substituted amino acid residues is 70% or more in the amino acid sequence in which at least one of the substitutions (A) to (AY) is introduced, and the relative activity of protein glutaminase at 65°C with respect to the activity of protein glutaminase at 60°C is improved as compared to the relative activity of the polypeptide consisting of the amino acid sequence set forth as SEQ ID NO: 1.

2. A DNA encoding the modified protein glutaminase according to claim 1.

3. An expression cassette or a recombinant vector comprising the DNA according to claim 2.

4. A transformant obtained by transforming a host using the expression cassette or recombinant vector according to claim 3.

5. A method for producing a modified protein glutaminase, comprising the step of culturing the transformant according to claim 4.

6. An enzyme agent comprising the modified protein glutaminase according to claim 1.

7. A modifier for a protein material, comprising the modified protein glutaminase according to claim 1.

8. A method for producing a modified protein material, comprising the step of applying the modified protein glutaminase according to claim 1 to a protein material.

FIG. 1

## INTERNATIONAL SEARCH REPORT

| | International application No. |
|---|---|
| | **PCT/JP2023/009183** |

### A. CLASSIFICATION OF SUBJECT MATTER

*C12N 15/57*(2006.01)i; *C12N 1/15*(2006.01)i; *C12N 1/19*(2006.01)i; *C12N 1/21*(2006.01)i; *C12N 5/10*(2006.01)i; *C12N 9/52*(2006.01)i; *C12N 9/80*(2006.01)i; *C12N 15/63*(2006.01)i; *C12P 1/00*(2006.01)i
FI: C12N15/57; C12N1/15; C12N1/19; C12N1/21; C12N5/10; C12N9/52; C12N9/80; C12N15/63 Z ZNA; C12P1/00 A

According to International Patent Classification (IPC) or to both national classification and IPC

### B. FIELDS SEARCHED

Minimum documentation searched (classification system followed by classification symbols)

C12N15/57; C12N1/15; C12N1/19; C12N1/21; C12N5/10; C12N9/52; C12N9/80; C12N15/63; C12P1/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

### C. DOCUMENTS CONSIDERED TO BE RELEVANT

| Category* | Citation of document, with indication, where appropriate, of the relevant passages | Relevant to claim No. |
|---|---|---|
| X | JP 5623282 B2 (AMANO ENZYME INC.) 12 November 2014 (2014-11-12) paragraphs [0010], [0037] | 1-8 |

☐ Further documents are listed in the continuation of Box C.    ☑ See patent family annex.

| | |
|---|---|
| \* Special categories of cited documents: <br> "A" document defining the general state of the art which is not considered to be of particular relevance <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
|---|---|
| **24 April 2023** | **16 May 2023** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

**INTERNATIONAL SEARCH REPORT**

International application No.

**PCT/JP2023/009183**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

   a. ☑ forming part of the international application as filed:

        ☑ in the form of an Annex C/ST.25 text file.

        ☐ on paper or in the form of an image file.

   b. ☐ furnished together with the international application under PCT Rule 13*ter*.1(a) for the purposes of international search only in the form of an Annex C/ST.25 text file.

   c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

   "In the form of an Annex C/ST.25 text file" above should be understood as "in ST.26 format".

Form PCT/ISA/210 (continuation of first sheet) (January 2015)

**INTERNATIONAL SEARCH REPORT**
Information on patent family members

International application No.

**PCT/JP2023/009183**

| Patent document cited in search report | Publication date (day/month/year) | Patent family member(s) | Publication date (day/month/year) |
|---|---|---|---|
| JP 5623282 B2 | 12 November 2014 | EP 2351837 A1 paragraphs [0010], [0037] | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- A novel protein-deamidating enzyme from Chryseobacterium proteolyticum sp. nov., a newly isolated bacterium from soil. *Applied and Environmental Microbiology*, 2000, vol. 66 (8), 3337-43

- **TATIANA A. TATSUSOVA** ; **THOMAS L. MADDEN**. *FEMS Microbiol. Lett.*, 1999, vol. 174, 247-250
- **SANGER et al.** *Proc. Natl. Acad. Sci.*, 1977, vol. 74, 5463